# EUROPEAN PATENT APPLICATION

(11) **EP 4 610 930 A1**
(43) Date of publication of application: **03.09.2025**
(21) Application number: 25160719.8
(22) Date of filing: 27.02.2025
(51) Int. Cl.: G06T 11/60

(54) **SYSTEMS AND METHODS FOR GENERATING A RADIOLOGY REPORT**

(30) Priority: 29.02.2024 US 202418592272
(71) Applicant: Tempus AI, Inc., Chicago, IL 60654 (US)
(72) Inventor: GOEL, Akshay, New York, New York (US); GANGWARE, Erika, Chicago, Illinois (US); HUNTER GORDON, Jacob William, West Orange, New Jersey (US); VENKATARAMAN, Jagadish, Menlo Park, California (US)
(74) Representative: Grund, Martin

(57) **Abstract**

System and methods for visualizing a cancer in a subject are provided herein. Medical images of a three-dimensional region of interest of the subject are obtained. The medical images are segmented by assigning labels corresponding to tissue types for each of a plurality of sets of one or more pixels in the medical images. Masks are generated for the tissue types based on the segmentation labels. A visual representation of tumor tissue is generated based on a corresponding mask for the tumor tissue. A visual representation of a skeleton of the subject is also generated based on the medical images. The visual representation of the tumor tissue and the visual representation of the skeleton of the subject is displayed in a single image, in a same spatial orientation as in the set of medical images, and at a same relative size as in the set of medical images.

## Description

### BACKGROUND

Medical imaging is routinely used in disease diagnosis and treatment. Hussain, S., et al., Modern Diagnostic Imaging Technique Applications and Risk Factors in the Medical Field: A Review, BioMed Research International, 2022:5164970 (2022), which is incorporated herein by reference in its entirety. However, manual interpretation of medical imaging is time consuming, expensive, and subjective. Moreover, radiology imaging is difficult to interpret for non-expert clinicians and patients. Radiology reports from trained radiologists can be time consuming to wait for and are often missing important quantitative measurements due to how time consuming it is to make such measurements.

### SUMMARY

Given the above background, what is needed in the art are improved methods and systems for generating a radiology report. As disclosed herein, the present disclosure provides improved systems and methods for generating a radiology report for interpretation by a clinician. Advantageously, the systems and methods described herein provide simple radiology reports with easily interpretable visuals of tumor burdens, e.g., evaluated from a CT scan and quantitative measurements thereof. In some embodiments, where longitudinal imaging data of a tumor is available, the systems and methods described herein further include visuals of the tumor from medical imaging and/or past quantitative measurements for a clear picture of how the patient's cancer is progressing, e.g., in view of ongoing treatment.

In one aspect, the disclosure provides a method for visualizing a cancer in a subject, at a computer system comprising one or more processors and a memory coupled to the one or more processors, the memory comprising one or more programs configured to be executed by the one or more processors.

In some embodiments, the method includes obtaining a first set of medical images of a three-dimensional region of interest of the subject, wherein the first set of medical images was collected at a first time using a first medical imaging modality, the three-dimensional region of interest of the subject comprises a plurality of tissue types, and the plurality of tissue types comprises a tumor tissue and a non-cancerous tissue of the subject.

In some such embodiments, the first set of medical images comprises a computed tomography (CT) scan of the three-dimensional region of the subject.

In some such embodiments, the cancer is a non-small cell lung cancer (NSCLC) and the plurality of tissue types includes non-cancerous lung tissue. In some such embodiments, the cancer is a pancreatic cancer and the plurality of tissue types includes non-cancerous pancreatic tissue. In some such embodiments, the plurality of tissue types includes lymph tissue. In some such embodiments, the plurality of tissue types includes vascular tissue.

In some embodiments, the method includes segmenting the set of medical images by assigning, for each respective set of one or more pixels in a first plurality of sets of one or more pixels in the set of medical images, a label corresponding to a respective tissue type in the plurality of tissue types based on one or more corresponding pixel values for the respective set of one or more pixels. In some embodiments, the method includes generating, for each respective tissue type in the plurality of tissue types, a corresponding mask based on respective sets of one or more pixels assigned the label corresponding to the respective tissue type.

The method includes generating a visual representation of the tumor tissue of the subject based on a corresponding mask for the tumor tissue. In some such embodiments, corresponding mesh surface is generated for the tumor tissue based on an outer boundary of the corresponding mask for the tumor tissue and smoothing edges of the corresponding mesh surface for the tumor tissue.

In some such embodiments, each respective set of one or more pixels in the first plurality of sets of one or more pixels corresponds to a respective voxel in a uniform three-dimensional grid of voxels defined for the set of medical images and for a respective tissue type in the plurality of tissue types, the corresponding mask comprises a binary indication, for each respective voxel in the three-dimensional grid of voxels, of whether the tissue represented in the voxel is the respective tissue type. In some such embodiments, the corresponding mask for the tumor tissue comprises a plurality of groups of non-zero voxels, wherein each respective group of non-zero voxels in the plurality of groups of non-zero voxels is separated from every other respective group of non-zero voxels in the plurality of groups of non-zero voxels by at least one zero voxel. In some such embodiments, generating the visual representation of the tumor tissue comprises generating a corresponding mesh surface for each respective group of non-zero voxels in the plurality of groups of non-zero voxels. In some such embodiments, the visual representation of the tumor tissue excludes representations of respective groups of non-zero voxels having a total volume below a first volume threshold.

In some such embodiments, the method also includes determining a volume for the cancer in the subject based on a volume contained within the mesh surface for the tumor tissue. In some such embodiments, the volume is determined after smoothing edges of the corresponding mesh surface for the tumor tissue.

In some embodiments, the method also includes generating a visual representation of a skeleton of the subject within the region of interest based on the first set of medical images.

In some such embodiments, this includes determining, for each respective set of one or more pixels in a second plurality of sets of one or more pixels in the set of medical images, a corresponding tissue density. In some such embodiments, a corresponding mask for the skeleton is generated based on respective sets of one or more pixels in the second plurality of sets of one or more pixels having a tissue density satisfying a set of one or more bone density criteria. In some such embodiments, the first set of medical images comprises a computed tomography (CT) scan and the set of one or more bone density criteria comprise a first criterion that is satisfied by a measure of central tendency for the pixel intensity of the respective set of one or more pixels of greater than a first threshold of from 300 to 500 Hounsfield units (HUs). In some such embodiments, the first set of medical images comprises a computed tomography (CT) scan and the set of one or more bone density criteria comprise a second criterion that is satisfied by a measure of central tendency for the pixel intensity of the respective set of one or more pixels of less than a second threshold of from 1750 to 2500 Hounsfield units (HUs).

In some such embodiments, a corresponding mesh surface for the skeleton is generated based on an outer boundary of the corresponding mask for the skeleton. In some such embodiments, edges of the corresponding mesh surface for the skeleton are smoothed.

In some such embodiments, each respective set of one or more pixels in the second plurality of sets of one or more pixels corresponds to a respective voxel in a second three-dimensional grid of voxels defined for the set of medical images and the corresponding mask for the skeleton comprises a binary indication, for each respective voxel in the second three-dimensional grid of voxels, of whether the tissue represented in the voxel is skeletal tissue. In some such embodiments, the visual representation of the skeleton excludes representations of respective groups of non-zero voxels in the three-dimensional grid of voxels having a total volume below a second volume threshold.

The method also includes displaying the visual representation of the tumor tissue, and optionally the visual representation of the skeleton of the subject in a single image, in a same spatial orientation as in the set of medical images, and at a same relative size as in the set of medical images.

In some such embodiments, the method also includes generating a report comprising the single image comprising the visual representation of the tumor tissue and the visual representation of the skeleton of the subject. In some such embodiments, the report further comprises one or more measurement for the cancerous tissue. In some such embodiments, the one or more measurement for the cancerous tissue comprises at least one measurement selected from a volume for the cancerous tissue, a cross-sectional length for the cancerous tissue, a density measurement for the cancerous tissue, a density for a non-cancerous tissue in the plurality of tissues, a distance from the cancerous tissue to a non-cancerous tissue in the plurality of tissues, and a distance from the cancerous tissue to a vascular structure in the three-dimensional region of interest of the subject. In some such embodiments, the report further comprises a change in a measurement for the cancerous tissue over time. In some such embodiments, the report further comprises, for each respective timepoint in a plurality of timepoints, a respective visual representation of the tumor tissue at the respective timepoint. In some such embodiments, the report further comprises a timeline indicating the timing of one or more events associated with the cancer in the subject. In some such embodiments, an event in the one or more events associated with the cancer in the subject is selected from an imaging examination of the cancer, a medical intervention for the cancer, a diagnosis for the cancer, a prognosis for the cancer, and a progression or regression of the cancer. In some such embodiments, the report further comprises a prognosis for the cancer in the subject. In some such embodiments, the report is displayed in a user interface on a second computer system comprising one or more processors, memory coupled to the one or more processors, and a display.

In some such embodiments, the single image further comprises a visual representation of a reference shape in the single image corresponding to a volume in the three-dimensional region of interest of the subject at a size of the same proportion to the visual representation of the tumor tissue and the visual representation of the skeleton of the subject relative to the set of medical images.

In some such embodiments, the single image further comprises a visual representation of a reference axial, coronal, or sagittal slice of the three-dimensional region of interest of the subject from the first set of medical images. In some such embodiments, the reference axial, coronal, or sagittal slice bisects the tumor tissue.

In some such embodiments, the single image comprises a three-dimensional representation of the tumor tissue and skeleton of the subject. In some such embodiments, the single image comprises a two-dimensional representation of the tumor tissue and skeleton of the subject.

In some such embodiments, the single image further comprises a visual representation of the non-cancerous tissue of the subject, in the same spatial orientation as in the set of medical images, and at a same relative size as in the set of medical images.

Note that the various embodiments described above can be combined with any other embodiments described herein. The features and advantages described in the specification are not all inclusive and, in particular, many additional features and advantages will be apparent to one of ordinary skill in the art in view of the drawings, specification, and claims. Moreover, it should be noted that the language used in the specification has been principally selected for readability and instructional purposes and may not have been selected to delineate or circumscribe the inventive subject matter.

### BRIEF DESCRIPTION OF THE DRAWINGS

In the drawings, embodiments of the systems and method of the present disclosure are illustrated by way of example. It is to be expressly understood that the description and drawings are only for the purpose of illustration and as an aid to understanding, and are not intended as a definition of the limits of the systems and methods of the present disclosure.
FIGS. 1A, 1B, and 1C illustrate a computer system for generating a radiology report, in accordance with some embodiments of the present disclosure.
FIGS. 2A, 2B, 2C, 2D, 2E, 2F, and 2G collectively provide a flowchart for an example method for generating a radiology report, in accordance with some embodiments of the present disclosure.
FIG. 3 illustrates a process for generating a radiology report, in accordance with some embodiments of the present disclosure.
FIG. 4 illustrates visual representations of a pancreatic tumor 402 and pancreas 404 generated from a CT scan, in accordance with some embodiments of the present disclosure.
FIG. 5 illustrates visual representations of a pancreatic tumor 502 and pancreas 504 generated from a CT scan, bisected with sagittal and coronal slices from the CT scan, in accordance with some embodiments of the present disclosure.
FIG. 6 illustrates visual representations of a pancreatic tumor 602, pancreas 604, and skeleton 606 generated from a CT scan, in accordance with some embodiments of the present disclosure.
FIG. 7 illustrates an example radiology report with visual representations of a non-small cell lung cancer (NSCLC) in context with the respiratory airway and skeleton at a first time point, as well as visual representation of the NSCLC over time, generated from CT scans, in accordance with some embodiments of the present disclosure.
FIG. 8 illustrates an example radiology report with visual representations of a pancreatic tumor in context with the pancreas and skeleton of the patient at a first time point, as well as visual representation of the pancreatic tumor over time, generated from CT scans, in accordance with some embodiments of the present disclosure.
FIG. 9 illustrates an example radiology report with visual representations of lung tumors in context with the skeleton of the patient, as well as a visual representation of the lung tumor islands alone, generated from a CT scan, in accordance with some embodiments of the present disclosure.
FIG. 10 illustrates 3-dimensional visual representations of a pancreatic tumor, pancreas, and vasculature surrounding the pancreas generated from a CT scan, as well as 2-dimensional representations of the pancreatic tumor, pancreas, and vasculature overlayed on axial slices from the CT scan at different depths, in accordance with some embodiments of the present disclosure.
FIG. 11 illustrates 3-dimensional visual representations of a pancreatic tumor, pancreas, and surrounding vasculature generated from a CT scan, in accordance with some embodiments of the present disclosure.

Like reference numerals refer to corresponding parts throughout the several views of the drawings.

### DETAILED DESCRIPTION

Reference will now be made in detail to embodiments, examples of which are illustrated in the accompanying drawings. In the following detailed description, numerous specific details are set forth in order to provide a thorough understanding of the present disclosure. However, it will be apparent to one of ordinary skill in the art that the present disclosure may be practiced without these specific details. In other instances, well-known methods, procedures, components, circuits, and networks have not been described in detail so as not to unnecessarily obscure aspects of the embodiments.

Oncologists have very limited time to prepare for appointments with cancer patients. However, radiology data is complicated, causing oncologists to spend extensive time before each appointment collecting and reviewing prior radiology scans and reports to interpret a patient's newest scans, e.g., to understand issues such as the trajectory of the cancer and effectiveness of current therapies, so they determine the best direction for future therapy. Advantageously, in some embodiments, the systems and methods described herein collect relevant data into a single display, e.g., a pdf or user interface, to aid clinicians saving significant time and expense associated with cancer therapy.

Moreover, patients often struggle understanding the results reported by their physician. Advantageously, in some embodiments, the systems and methods described herein contextualizes that information into easy to understand visuals alongside information from the patient's journey into a report the patient retain after their visit.

### Definitions

As used herein, the term "measure of central tendency" refers to a central or representative value for a distribution of values. Non-limiting examples of measures of central tendency include an arithmetic mean, weighted mean, midrange, midhinge, trimean, geometric mean, geometric median, Winsorized mean, median, and mode of the distribution of values.

As used herein, the term "subject" refers to any living or non-living organism including, but not limited to, a human *(e.g.,* a male human, female human, fetus, pregnant female, child, or the like), a non-human mammal, or a non-human animal. Any human or non-human animal can serve as a subject, including but not limited to mammal, reptile, avian, amphibian, fish, ungulate, ruminant, bovine *(e.g.,* cattle), equine *(e.g.,* horse), caprine and ovine *(e.g.,* sheep, goat), swine *(e.g.,* pig), camelid *(e.g.,* camel, llama, alpaca), monkey, ape *(e.g.,* gorilla, chimpanzee), ursid *(e.g.,* bear), poultry, dog, cat, mouse, rat, fish, dolphin, whale and shark. In some embodiments, a subject is a male or female of any age *(e.g.,* a man, a woman, or a child).

As used herein the term "cancer," "cancerous tissue," or "tumor" refers to an abnormal mass of tissue in which the growth of the mass surpasses, and is not coordinated with, the growth of normal tissue. A cancer or tumor can be defined as "benign" or "malignant" depending on the following characteristics: degree of cellular differentiation including morphology and functionality, rate of growth, local invasion and metastasis. A "benign" tumor can be well differentiated, have characteristically slower growth than a malignant tumor and remain localized to the site of origin. In addition, in some cases a benign tumor does not have the capacity to infiltrate, invade or metastasize to distant sites. A "malignant" tumor can be a poorly differentiated (anaplasia), have characteristically rapid growth accompanied by progressive infiltration, invasion, and destruction of the surrounding tissue. Furthermore, a malignant tumor can have the capacity to metastasize to distant sites. Accordingly, a cancer cell is a cell found within the abnormal mass of tissue whose growth is not coordinated with the growth of normal tissue. Accordingly, a "tumor sample" refers to a biological sample obtained or derived from a tumor of a subject, as described herein.

As used herein, the term "classification" can refer to any number(s) or other characters(s) that are associated with a particular property of a sample. For example, in some embodiments, the term "classification" can refer to a tissue type in a subject, e.g., a tissue type determined from a set of medical images such as a CT scan. The term classification can also refer to a type of cancer in a subject, a stage of cancer in a subject, a prognosis for a cancer in a subject, a tumor load, a presence of tumor metastasis in a subject, and the like. The classification can be binary (e.g., positive or negative) or have more levels of classification (*e.g.*, a scale from 1 to 10 or 0 to 1). The terms "cutoff" and "threshold" can refer to predetermined numbers used in an operation. For example, a cutoff density can refer to a density above which tissue is excluded from being classified as skeletal tissue. A threshold value can be a value above or below which a particular classification applies. Either of these terms can be used in either of these contexts.

As used herein, an "effective amount" or "therapeutically effective amount" is an amount sufficient to affect a beneficial or desired clinical result upon treatment. An effective amount can be administered to a subject in one or more doses. In terms of treatment, an effective amount is an amount that is sufficient to palliate, ameliorate, stabilize, reverse or slow the progression of the disease, or otherwise reduce the pathological consequences of the disease. The effective amount is generally determined by the physician on a case-by-case basis and is within the skill of one in the art. Several factors are typically taken into account when determining an appropriate dosage to achieve an effective amount. These factors include age, sex and weight of the subject, the condition being treated, the severity of the condition and the form and effective concentration of the therapeutic agent being administered.

The terminology used in the present disclosure is for the purpose of describing particular embodiments only and is not intended to be limiting of the invention. As used in the description of the invention and the appended claims, the singular forms "a", "an" and "the" are intended to include the plural forms as well, unless the context clearly indicates otherwise. It will also be understood that the term "and/or" as used herein refers to and encompasses any and all possible combinations of one or more of the associated listed items. It will be further understood that the terms "comprises" and/or "comprising," when used in this specification, specify the presence of stated features, integers, steps, operations, elements, and/or components, but do not preclude the presence or addition of one or more other features, integers, steps, operations, elements, components, and/or groups thereof. Furthermore, to the extent that the terms "including," "includes," "having," "has," "with," or variants thereof are used in either the detailed description and/or the claims, such terms are intended to be inclusive in a manner similar to the term "comprising."

As used herein, the term "if" may be construed to mean "when" or "upon" or "in response to determining" or "in response to detecting," depending on the context. Similarly, the phrase "if it is determined" or "if [a stated condition or event] is detected" may be construed to mean "upon determining" or "in response to determining" or "upon detecting [the stated condition or event]" or "in response to detecting [the stated condition or event]," depending on the context.

It will also be understood that, although the terms first, second, *etc.* may be used herein to describe various elements, these elements should not be limited by these terms. These terms are only used to distinguish one element from another. For example, a first subject could be termed a second subject, and, similarly, a second subject could be termed a first subject, without departing from the scope of the present disclosure. The first subject and the second subject are both subjects, but they are not the same subject. Furthermore, the terms "subject," "user," and "patient" are used interchangeably herein.

Reference will now be made in detail to embodiments, examples of which are illustrated in the accompanying drawings. In the following detailed description, numerous specific details are set forth in order to provide a thorough understanding of the present disclosure, including example systems, methods, techniques, instruction sequences, and computing machine program products that embody illustrative implementations. However, the illustrative discussions below are not intended to be exhaustive or to limit the implementations to the precise forms disclosed. Many modifications and variations are possible in view of the above teachings. The features described herein are not limited by the illustrated ordering of acts or events, as some acts can occur in different orders and/or concurrently with other acts or events.

The implementations provided herein are chosen and described in order to best explain the principles and their practical applications, to thereby enable others skilled in the art to best utilize the various embodiments with various modifications as are suited to the particular use contemplated. In some instances, well-known methods, procedures, components, circuits, and networks have not been described in detail so as not to unnecessarily obscure aspects of the embodiments. In other instances, it will be apparent to one of ordinary skill in the art that the present disclosure may be practiced without one or more of the specific details.

It will be appreciated that, in the development of any such actual implementation, numerous implementation-specific decisions are made in order to achieve the designer's specific goals, such as compliance with use case- and business-related constraints, and that these specific goals will vary from one implementation to another and from one designer to another. Moreover, it will be appreciated that though such a design effort might be complex and time-consuming, it will nevertheless be a routine undertaking of engineering for those of ordering skill in the art having the benefit of the present disclosure.

### Example System Embodiments

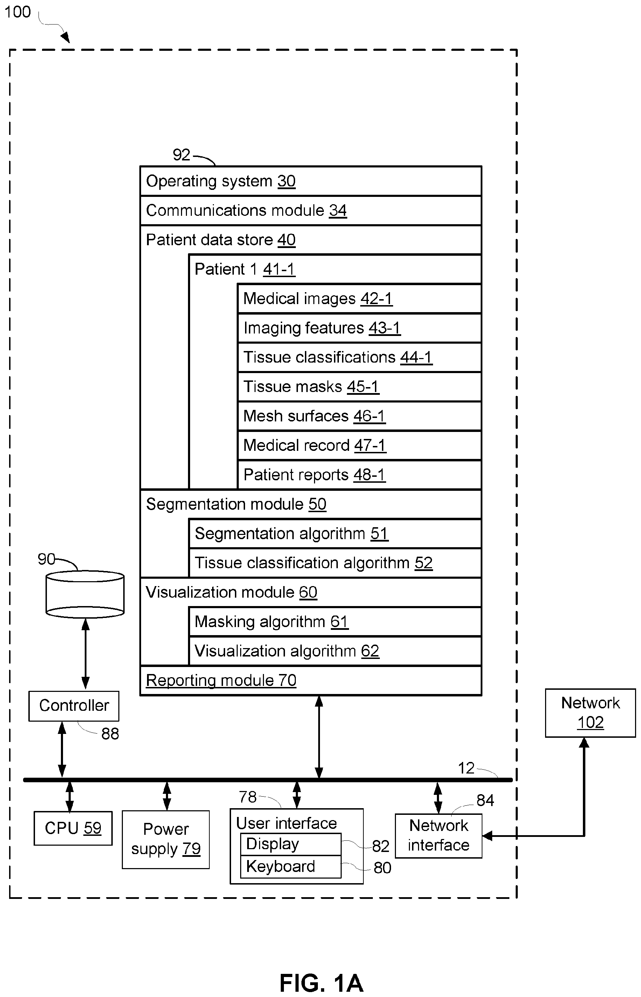

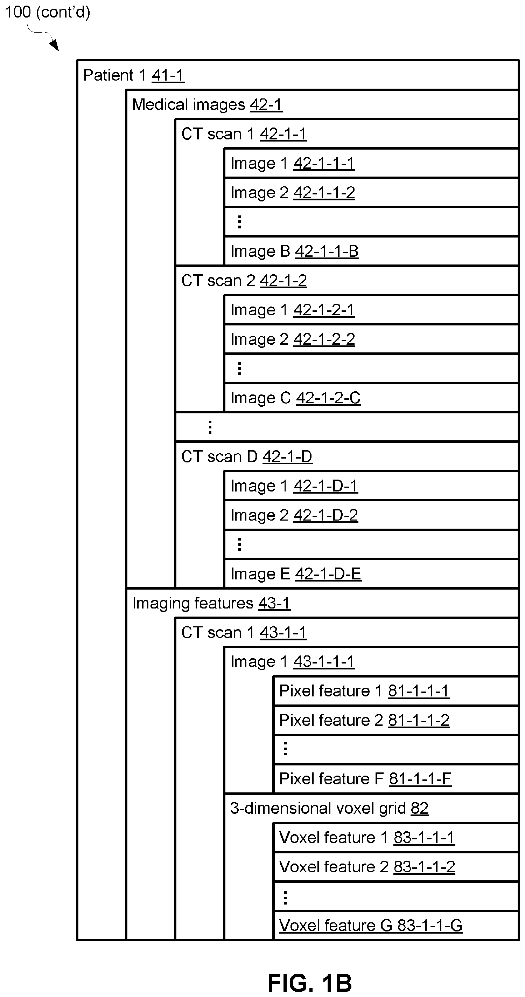

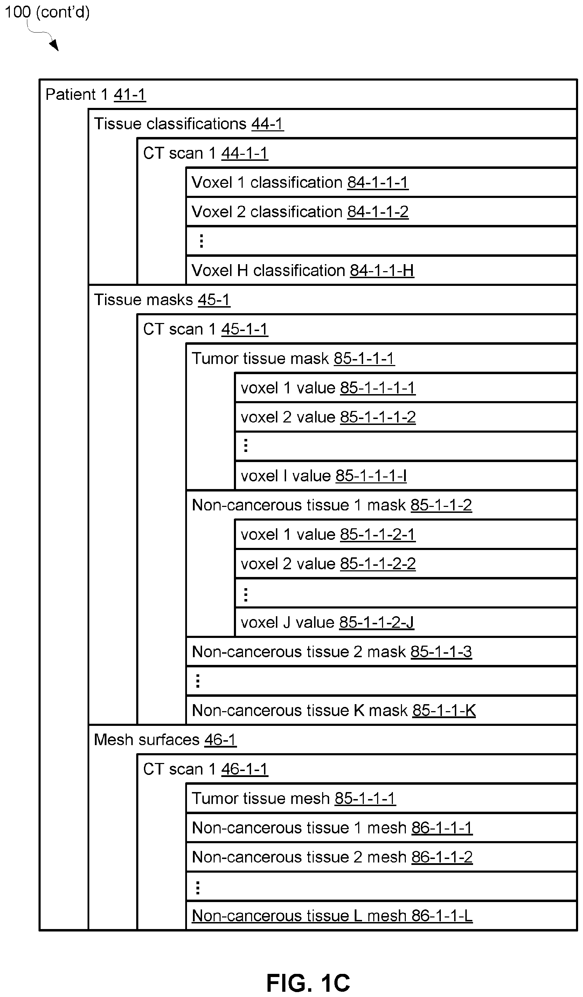

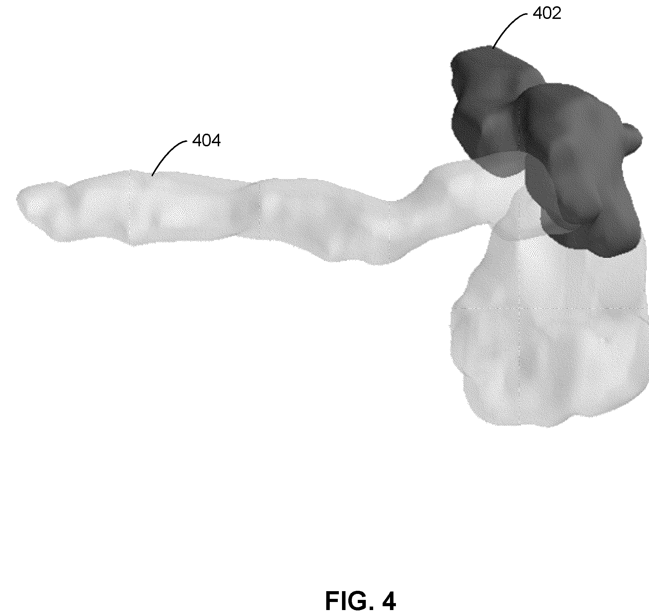

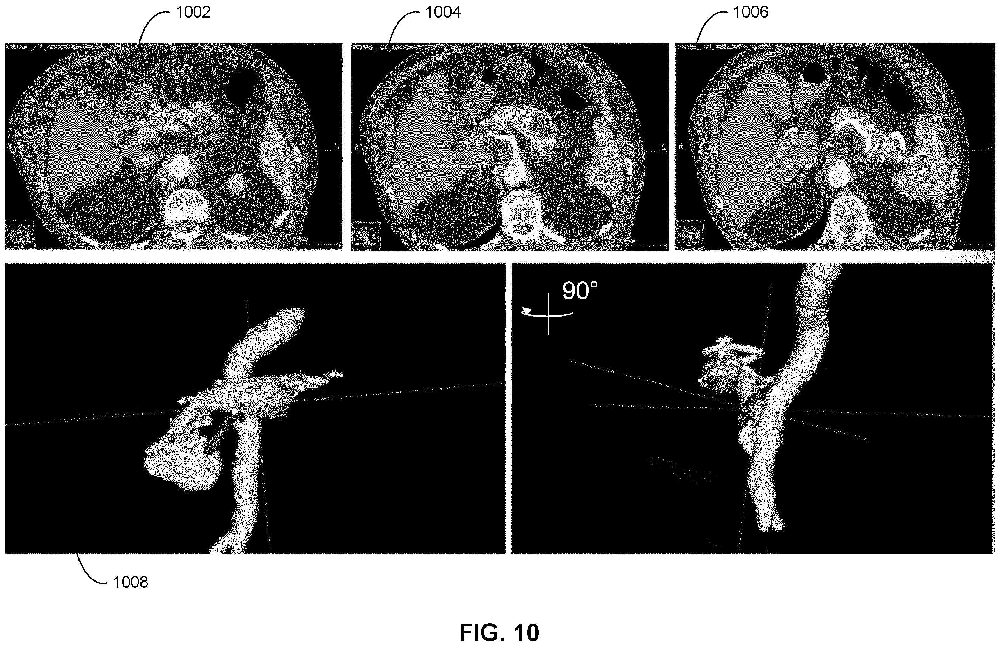

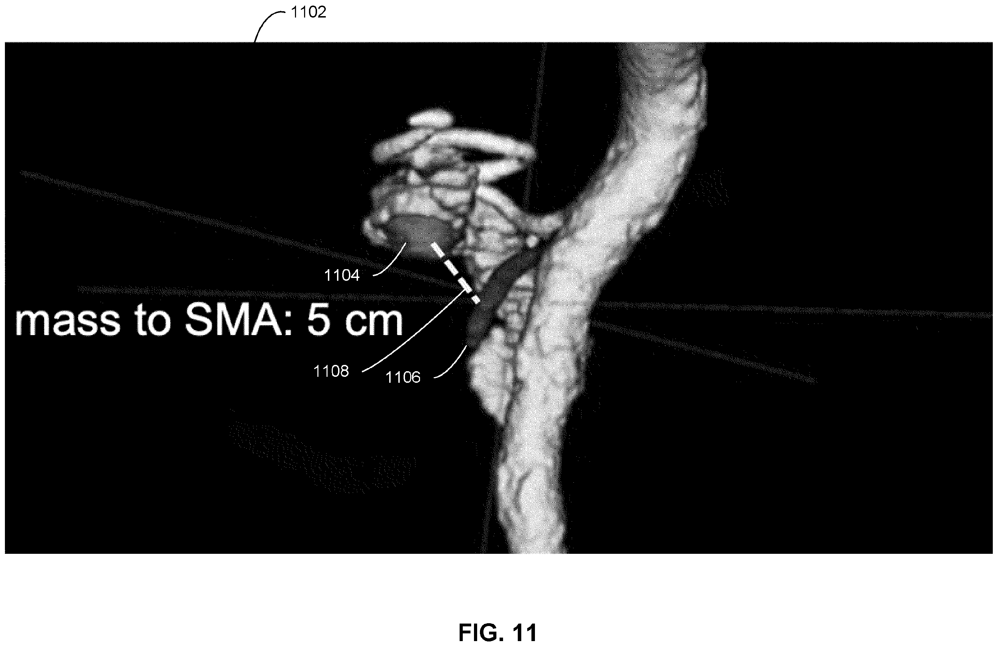

Improved systems and methods for generating a radiology report are described herein. Example details of such systems, devices, and/or processes in accordance with the present disclosure are described below in relation to FIGS. 1-3. Examples of the products of these systems, devices, and/or processes are further described with respect to FIGS. 4-11.

FIGS. 1A-1C collectively illustrate a computer system for generating a radiology report, in accordance with some embodiments of the present disclosure. In typical embodiments, computer system 100 comprises one or more computers. For purposes of illustration in FIG. 1A, the computer system 100 is represented as a single computer that includes all of the functionality of the disclosed computer system 100. However, the present disclosure is not so limited. The functionality of the computer system 100 may be spread across any number of networked computers and/or reside on each of several networked computers and/or virtual machines. One of skill in the art will appreciate that a wide array of different computer topologies are possible for the computer system 100 and all such topologies are within the scope of the present disclosure.

Turning to FIG. 1A with the foregoing in mind, the computer system 100 comprises one or more processing units (CPUs) 59, a network or other communications interface 84, a user interface 78 (e.g., including an optional display 82 and optional keyboard 80 or other form of input device), a memory 92 *(e.g.,* random access memory, persistent memory, or combination thereof), one or more magnetic disk storage and/or persistent devices 90 optionally accessed by one or more controllers 88, one or more communication busses 12 for interconnecting the aforementioned components, and a power supply 79 for powering the aforementioned components. To the extent that components of memory 92 are not persistent, data in memory 92 can be seamlessly shared with non-volatile memory 90 or portions of memory 92 that are non-volatile or persistent using known computing techniques such as caching. Memory 92 and/or memory 90 can include mass storage that is remotely located with respect to the central processing unit(s) 59. In other words, some data stored in memory 92 and/or memory 90 may in fact be hosted on computers that are external to computer system 100 but that can be electronically accessed by the computer system 100 over network 102 (e.g., an Internet, intranet, or other form of network or electronic cable) using network interface 84. In some embodiments, the computer system 100 makes use of models that are run from the memory associated with one or more graphical processing units in order to improve the speed and performance of the system. In some alternative embodiments, the computer system 100 makes use of models that are run from memory 92 rather than memory associated with a graphical processing unit.

The memory 92 of the computer system 100 stores:
- an operating system 30 that includes procedures for handling various basic system services;
- a communication module 34, which connects to and communicates with other network devices (e.g., a local network, such as a router that provides Internet connectivity, networked storage devices, network routing devices, server systems, other computer systems 100, and/or other connected devices) coupled to one or more communication networks via the network interface 84 (e.g., wired or wireless);
- a patient data store 40, including one or more types of medical data and/or output from the methods described herein, for one or more patients 41. In some embodiments, the medical data includes one or more types of data selected from:
   ∘ one or more medical images 42 of a region of interest in a subject with cancer. In some embodiments, the medical images comprise multiple images of a region of interest in a subject collected at a single time point that represent a three-dimensional volume in the subject. For example, FIG. 1B illustrates a medical image store 42-1 containing a series of CT scans 42-1-i (42-1-1, ..., 42-1-D) acquired at different times for a patient 41-1, where each CT scan 42-1-i includes a plurality of image slices 42-1-1-j (e.g., 42-1-1-1, ..., 42-1-1-B for CT scan 42-1-1) of a three-dimensional region of interest in the subject.
   ∘ imaging features 43 extracted from medical image set of a region of interest in a subject with cancer (e.g., medical images 42). In some embodiments, such features may be determined at the pixel level, e.g., pixel features 81-1-1-1, ..., 81-1-1-F for image 43-1-1-1 of CT scan 43-1-1 as illustrated in Figure 1B, or the voxel level, e.g., voxel features 83-1-1-1, ..., 83-1-1-F for voxel grid 82 established for image 43-1-1-1 of CT scan 43-1-1 as illustrated in Figure 1B.
   ∘ tissue classifications 44 for one or more medical image sets, generated by analyzing imaging features 43 using a segmentation module 50. For example, as illustrated in Figure 1C, tissue classifications 44-1 provide a classification of the tissue type in each voxel 84-1-1-i in 3-dimensional voxel grid 82 by evaluating voxel features 83-1-1-i using segmentation module 50.
   ∘ tissue masks 45 of one or more imaged tissues, e.g., generated based on tissue classifications 44. For example, FIG. 1C illustrates tumor tissue mask 85-1-1-1 and non-cancerous tissue masks 85-1-1-2, ..., 85-1-1-K generated from voxel classifications 83-1-1-1, ...,83-1-1-H. In some embodiments, the tissue masks 85 include a value 85-i-j-k-l for each voxel in a three-dimensional voxel grid established for a region of interest in a subject indicating whether the tissue represented in the voxel is that type of tissue. For example, as illustrated in Figure 1C, tumor tissue mask 85-1-1-1 includes voxel values 85-1-1-1-1, ..., 85-1-1-1-I. In some embodiments, the voxel value is a binary value, e.g., either 1 (e.g., indicating the tissue represented by the voxel is of the type of tissue represented by the mask) or 0 (e.g., indicating that the tissue represented by the voxel is not of the type of tissue represented by the mask).
   ∘ mesh surfaces 46 for visualizing one or more tissue types imaged in a subject with cancer, e.g., generated by processing tissue masks 45 using a visualization module 60,
   ∘ medical records 47 for one or more patients, and
   ∘ patient reports 48 generated by analyzing imaging data, e.g., medical images 42, and optionally other medical information, e.g., medical records 47;
- a segmentation module 50, including a segmentation algorithm 61 and/or tissue classification algorithm 62 for segmenting different tissues in medical images (e.g., medical images 42) and/or determining tissue type for such tissues, e.g., for further visualization of cancerous and/or non-cancerous tissues in a subject with cancer;
- a visualization module 60, including a masking algorithm 61 and/or visualization algorithm 62 for generating visual representations of cancerous and/or non-cancerous tissues identified in medical images (e.g., medical images 42); and
- reporting module 70 for generating a report including a visual representation of one or more cancerous tissue and/or non-cancerous tissue (e.g., based on mesh surfaces 46) identified in medical images (e.g., medical images 42), optionally including other biographical and/or medical information about the subject, e.g., from medical records 47.

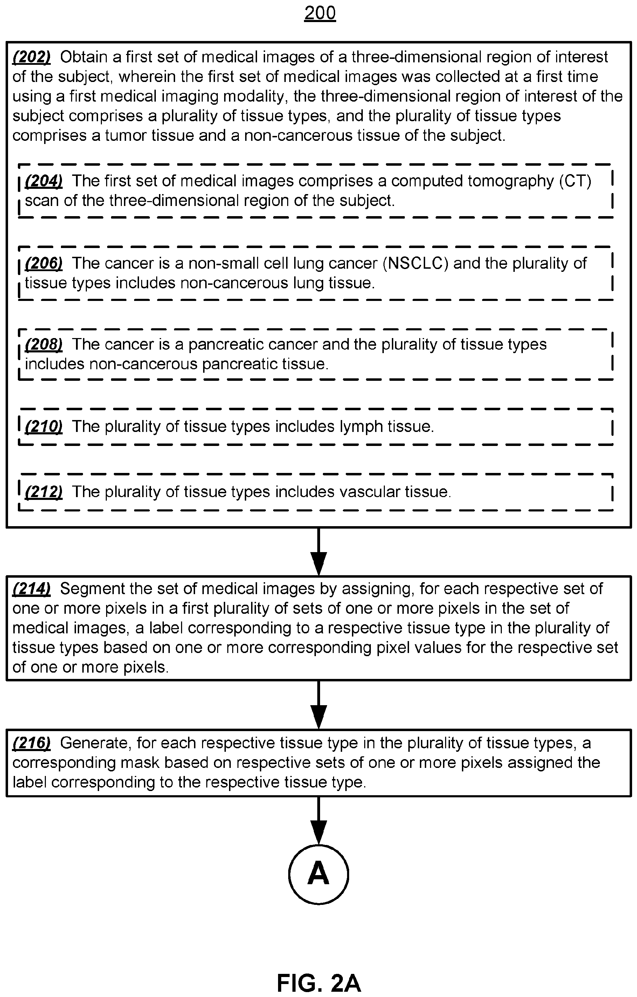

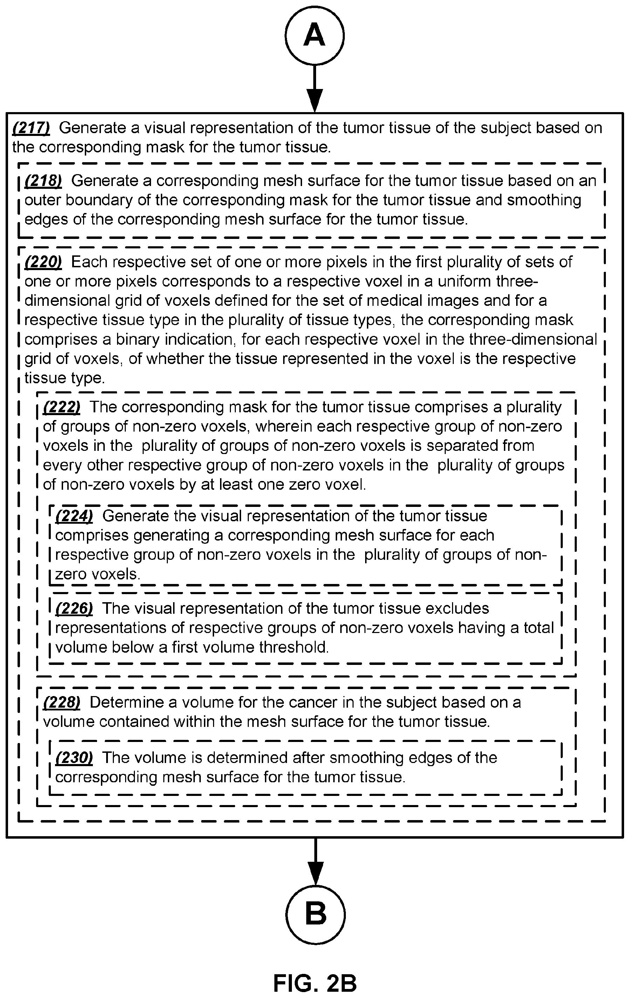

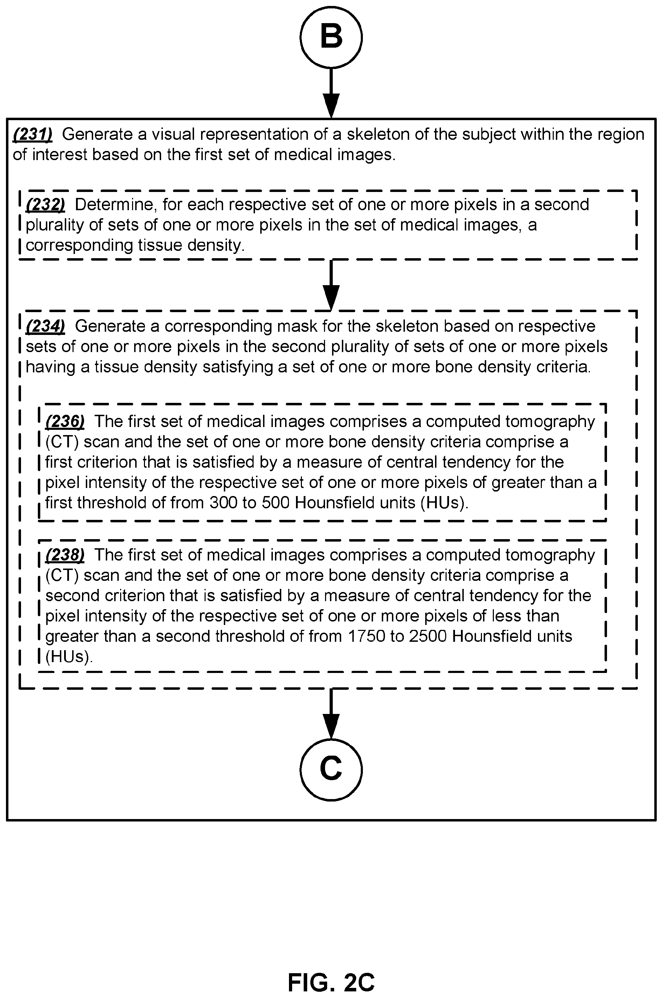

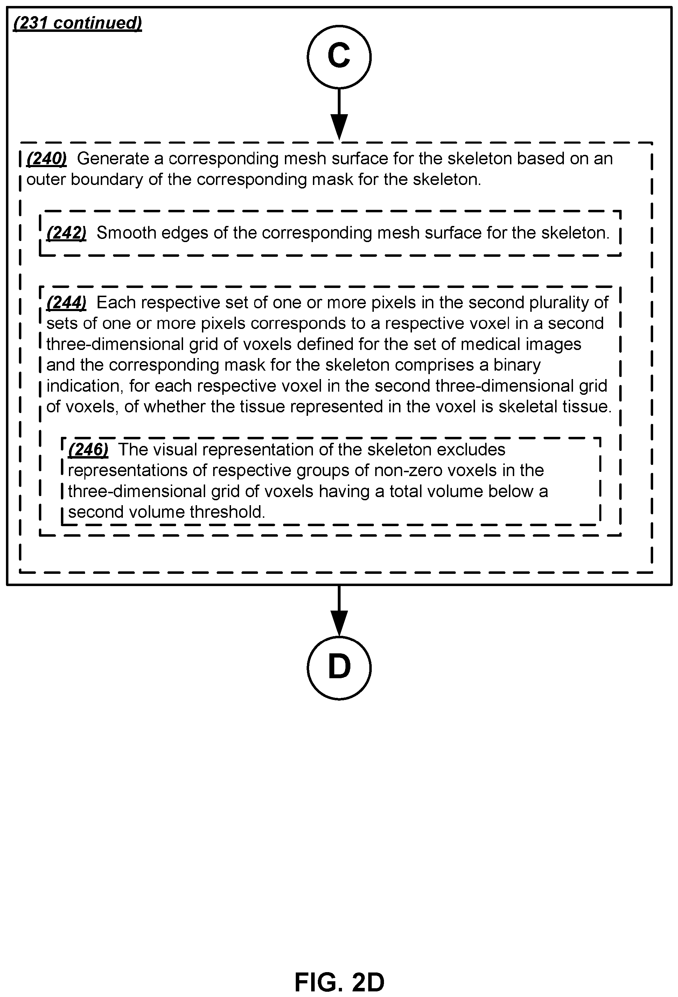

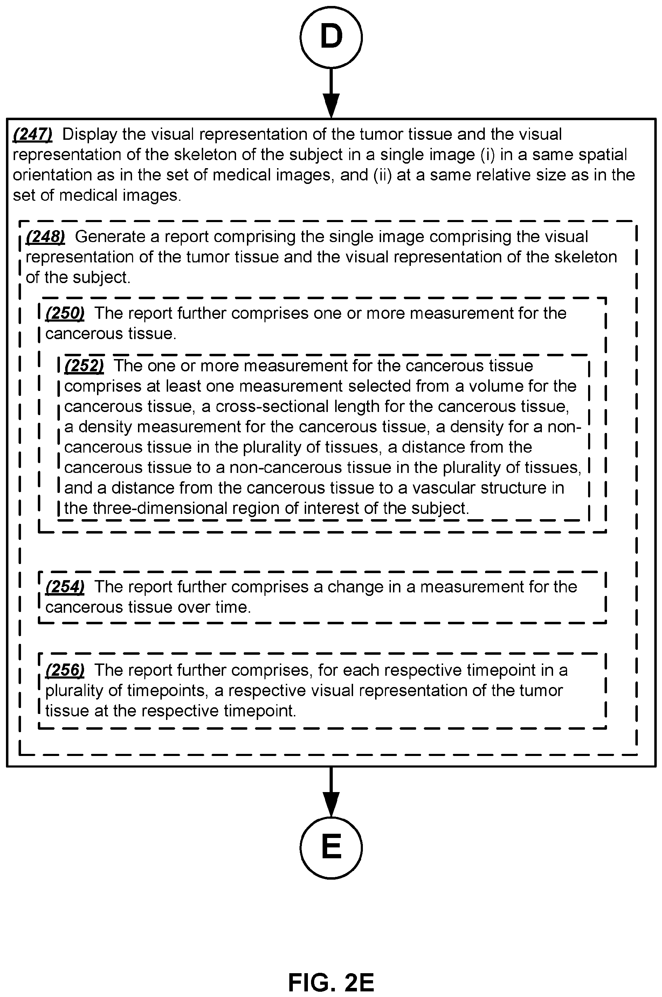

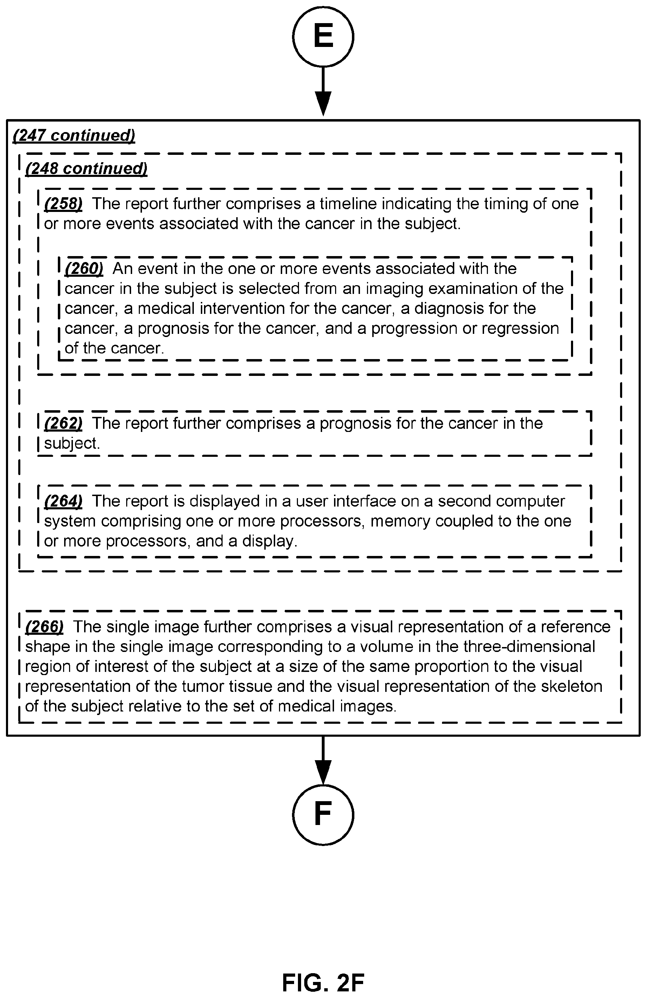

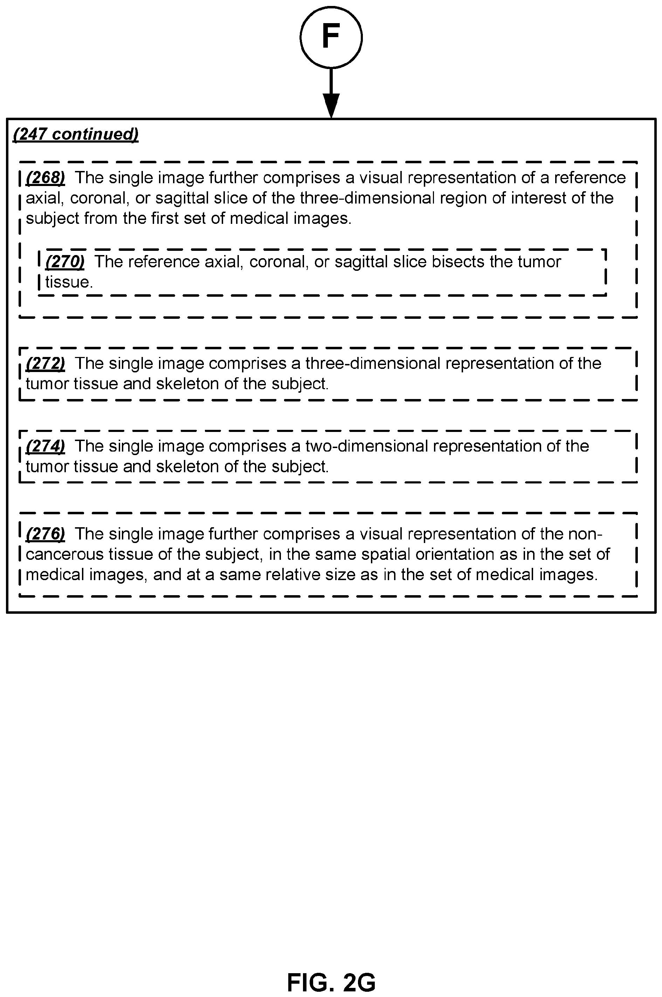

In some embodiments, one or more of the above identified data elements or modules of the computer system 100 are stored in one or more of the previously mentioned memory devices, and correspond to a set of instructions for performing a function described above. The above identified data, modules or programs (*e.g*., sets of instructions) need not be implemented as separate software programs, procedures or modules, and thus various subsets of these modules may be combined or otherwise re-arranged in various implementations. In some implementations, the memory 92 and/or 90 optionally stores a subset of the modules and data structures identified above. Furthermore, in some embodiments the memory 92 and/or 90 stores additional modules and data structures not described above. Details of the modules and data structures identified above are further described below in reference to FIGS. 2-11.

FIGS. 2A-2K collectively provide a flowchart for an example method 200 for generating a radiology report, in accordance with some embodiments. In some embodiments, the method 200 is performed at a computer system 100 that includes one or more processors (e.g., CPU 59) and memory (e.g., memory 90 or memory 92). In some embodiments, a user initiates, at the computer system 100, an instruction to start the process for generating a radiology report. In some embodiments, the computer system 100 performs the steps as noted in FIG. 2.

Referring to block 202, in some embodiments, the method includes obtaining a first set of medical images of a three-dimensional region of interest of the subject, wherein the first set of medical images was collected at a first time using a first medical imaging modality, the three-dimensional region of interest of the subject comprises a plurality of tissue types, and the plurality of tissue types comprises a tumor tissue and a non-cancerous tissue of the subject. In some embodiments, the set of medical images are cross-sectional imaging scans of the subject, such as CT scans, PET scans, or hybrids thereof. For a review on CT and PET medical imaging see, for example, Al-Sharify, Z.T., et al., "A critical review on medical imaging techniques (CT and PET scans) in the medical field," OP Conf. Ser.: Mater. Sci. Eng., 870:012043 (2020), the content of which is incorporated herein by reference.

In some embodiments, the obtaining is performed by a system 100 in response to receiving a user input requesting a report for a cancer in the subject. In some embodiments, the system queries electronic medical data, e.g., specific to the subject (e.g., a personal electronic health record) or generic to medical imaging datasets associated with a medical entity (e.g., healthcare provider, clinic, hospital, hospital system, medical insurer, etc.), for a most recent medical imaging dataset associated with a cancer. In some embodiments, the system further queries electronic medical data to determine whether earlier medical imaging data for the same cancer is also available.

Referring to block 204, in some embodiments, the first set of medical images comprises a computed tomography (CT) scan of the three-dimensional region of the subject. In in some embodiments, the medical imaging dataset comprises a magnetic resonance imaging (MRI) dataset. Generally, CT scans include from 4 to 640 individual images, each corresponding to a different plane in a three-dimensional region of the subject. The images are collected on a detector, commonly a flat panel, Charge Coupled Device (CCD), or active-pixel sensor (APS) such as a complementary metal-oxide-semiconductors (CMOS) or scientific CMOS (sCMOS) image sensor. Commonly, the detector comprises a 1000 x 1000 to 3000 x 3000 pixel array, although arrays with larger or smaller pixel arrays may find use in the systems and methods described herein.

However, the methods and systems described herein are not limited by the use of CT scans. In fact, many imaging modalities may be used in these methods and systems. For a review of various imaging modalities that can be used to visualize tumors in a subject see, for example, Rowe SP, and Pomper MG., "Molecular imaging in oncology: Current impact and future directions," CA Cancer J Clin. (2021), the disclosure of which is incorporated herein by reference.

In some embodiments, the medical imaging data comprises a plurality of X-ray image sets, a plurality of computed tomography (CT) image sets, a plurality of positron emission tomography (PET) image sets, a plurality of magnetic resonance imaging (MRI) image sets, a plurality of single-photon emission computed tomography (SPECT) image sets, a plurality of mammography image sets, a plurality of sonography (ultrasound) image sets, or a combination thereof. For a review of medical imaging techniques used in diagnostics see, for example, Hussain, S., et al., Modern Diagnostic Imaging Technique Applications and Risk Factors in the Medical Field: A Review, BioMed Research International, 2022:5164970 (2022), which is incorporated herein by reference in its entirety.

Many of the medical imaging modalities used in diagnostics generate datasets made up of multiple images. For example, CT scans use a rotating x-ray tube to take multiple images at different angles which can be processed to generate 2-dimensional or 3-dimensional tomographic images of a region of interest. Depending on the specific implementation and region of interest being imaged, CT scans can generate tens to thousands of images. Similarly, MRI scans acquire image slices of a region of interest. Depending on the specific implementation and region of interest being imaged, MRI scans can generate tens to thousands of images.

In some embodiments, the medical imaging dataset comprises an ultrasound dataset. For discussion of the use of ultrasound imaging in clinical cancer therapy see, for example, Sarikaya, I., "Biology of Cancer and PET Imaging: Pictorial Review," J. Nucl. Med. Technol., 16(1):531 (2022), the disclosure of which is incorporated herein by reference. In some embodiments, the medical imaging dataset includes a position emission tomography (PET) dataset. For discussion of the use of PET imaging in clinical cancer therapy see, for example, Diaz-Alejo J.F., et. al., "Ultrasounds in cancer therapy: A summary of their use and unexplored potential," Oncol. Rev., 50(2):81-89 (2022), the disclosure of which is incorporated herein by reference. In some embodiments, the medical imaging dataset comprises an X-ray dataset.

In some embodiments, the first set of medical images has at least 8 images. In some embodiments, the first set of medical images has at least 32 images. In some embodiments, the first set of medical images has at least 128 images. In some embodiments, the first set of medical images has at least 4 images, at least 8 images, at least 16 images, at least 32 images, at least 64 images, at least 128 images, at least 256 images, at least 320 images, at least 640, at least 1280 images, at least 2560 images or more. In some embodiments, the first set of medical images has no more than 100,000 images. In some embodiments, the first set of medical images has no more than 50,000 images. In some embodiments, the first set of medical images has no more than 10,000 images. In some embodiments, the first set of medical images has no more than 2500 images. In some embodiments, the first set of medical images has no more than 1500 images.

In some embodiments, the first set of medical images has from 4 to 2560 images. In some embodiments, the first set of medical images has from 4 to 1280 images. In some embodiments, the first set of medical images has from 4 to 640 images. In some embodiments, the first set of medical images has from 4 to 320 images. In some embodiments, the first set of medical images has from 4 to 256 images. In some embodiments, the first set of medical images has from 4 to 128 images. In some embodiments, the first set of medical images has from 4 to 64 images. In some embodiments, the first set of medical images has from 8 to 2560 images. In some embodiments, the first set of medical images has from 8 to 1280 images. In some embodiments, the first set of medical images has from 8 to 640 images. In some embodiments, the first set of medical images has from 8 to 320 images. In some embodiments, the first set of medical images has from 8 to 256 images. In some embodiments, the first set of medical images has from 8 to 128 images. In some embodiments, the first set of medical images has from 8 to 64 images. In some embodiments, the first set of medical images has from 16 to 2560 images. In some embodiments, the first set of medical images has from 16 to 1280 images. In some embodiments, the first set of medical images has from 16 to 640 images. In some embodiments, the first set of medical images has from 16 to 320 images. In some embodiments, the first set of medical images has from 16 to 256 images. In some embodiments, the first set of medical images has from 16 to 128 images. In some embodiments, the first set of medical images has from 16 to 64 images. In some embodiments, the first set of medical images has from 64 to 2560 images. In some embodiments, the first set of medical images has from 64 to 1280 images. In some embodiments, the first set of medical images has from 64 to 640 images. In some embodiments, the first set of medical images has from 64 to 320 images. In some embodiments, the first set of medical images has from 64 to 256 images. In some embodiments, the first set of medical images has from 64 to 128 images. In some embodiments, the first set of medical images has from 128 to 2560 images. In some embodiments, the first set of medical images has from 128 to 1280 images. In some embodiments, the first set of medical images has from 128 to 640 images. In some embodiments, the first set of medical images has from 128 to 320 images. In some embodiments, the first set of medical images has from 128 to 256 images. In some embodiments, the first set of medical images has from 256 to 2560 images. In some embodiments, the first set of medical images has from 256 to 1280 images. In some embodiments, the first set of medical images has from 256 to 640 images. In some embodiments, the first set of medical images has from 256 to 320 images.

In some embodiments, an image in the set of medical images has at least 250,000 pixels. In some embodiments, an image in the set of medical images has at least 1 million pixels. In some embodiments, an image in the set of medical image has at least 2.5 million pixels. In some embodiments, an image in the set of medical images has at least 5 million pixels. In some embodiments, an image in the set of medical images has no more than 1 billion pixels. In some embodiments, an image in the set of medical images has no more than 100 million pixels. In some embodiments, an image in the set of medical images has no more than 10 million pixels. In some embodiments, an image in the set of medical images has no more than 5 million pixels.

In some embodiments, an image in the set of medical images has from 250,000 to 1 billion pixels. In some embodiments, an image in the set of medical images has from 250,000 to 100 million pixels. In some embodiments, an image in the set of medical images has from 250,000 to 10 million pixels. In some embodiments, an image in the set of medical images has from 250,000 to 5 million pixels. In some embodiments, an image in the set of medical images has from 1 million to 1 billion pixels. In some embodiments, an image in the set of medical images has from 1 million to 100 million pixels. In some embodiments, an image in the set of medical images has from 1 million to 10 million pixels. In some embodiments, an image in the set of medical images has from 1 million to 5 million pixels. In some embodiments, an image in the set of medical images has from 2.5 million to 1 billion pixels. In some embodiments, an image in the set of medical images has from 2.5 million to 100 million pixels. In some embodiments, an image in the set of medical images has from 2.5 million to 10 million pixels. In some embodiments, an image in the set of medical images has from 2.5 million to 5 million pixels.

In some embodiments, the first set of medical images has at least 5 million pixels. In some embodiments, the first set of medical images has at least 10 million pixels. In some embodiments, the first set of medical images has at least 50 million pixels. In some embodiments, the first set of medical images has at least 100 million pixels. In some embodiments, the first set of medical images has at least 250 million pixels. In some embodiments, the first set of medical images has at least 500 million pixels. In some embodiments, the first set of medical images has at least 1 billion pixels. In some embodiments, the first set of medical images has no more than 100 billion pixels. In some embodiments, the first set of medical images has no more than 50 billion pixels. In some embodiments, the first set of medical images has no more than 10 billion pixels. In some embodiments, the first set of medical images has no more than 5 billion pixels. In some embodiments, the first set of medical images has no more than 1 billion pixels.

In some embodiments, the first set of medical images has from 5 million to 100 billion pixels. In some embodiments, the first set of medical images has from 5 million to 50 billion pixels. In some embodiments, the first set of medical images has from 5 million to 10 billion pixels. In some embodiments, the first set of medical images has from 5 million to 5 billion pixels. In some embodiments, the first set of medical images has from 5 million to 1 billion pixels. In some embodiments, the first set of medical images has from 10 million to 100 billion pixels. In some embodiments, the first set of medical images has from 10 million to 50 billion pixels. In some embodiments, the first set of medical images has from 10 million to 10 billion pixels. In some embodiments, the first set of medical images has from 10 million to 5 billion pixels. In some embodiments, the first set of medical images has from 10 million to 1 billion pixels. In some embodiments, the first set of medical images has from 50 million to 100 billion pixels. In some embodiments, the first set of medical images has from 50 million to 50 billion pixels. In some embodiments, the first set of medical images has from 50 million to 10 billion pixels. In some embodiments, the first set of medical images has from 50 million to 5 billion pixels. In some embodiments, the first set of medical images has from 50 million to 1 billion pixels. In some embodiments, the first set of medical images has from 100 million to 100 billion pixels. In some embodiments, the first set of medical images has from 100 million to 50 billion pixels. In some embodiments, the first set of medical images has from 100 million to 10 billion pixels. In some embodiments, the first set of medical images has from 100 million to 5 billion pixels. In some embodiments, the first set of medical images has from 100 million to 1 billion pixels. In some embodiments, the first set of medical images has from 250 million to 100 billion pixels. In some embodiments, the first set of medical images has from 250 million to 50 billion pixels. In some embodiments, the first set of medical images has from 250 million to 10 billion pixels. In some embodiments, the first set of medical images has from 250 million to 5 billion pixels. In some embodiments, the first set of medical images has from 250 million to 1 billion pixels.

In some embodiments, the plurality of non-cancerous tissues includes an organ tissue that is proximal to the tumor. In some embodiments, the organ tissue will be the tissue of origin for the case. For example, referring to block 206, in some embodiments, the cancer is a non-small cell lung cancer (NSCLC) and the plurality of tissue types includes non-cancerous lung tissue. Similarly, referring to block 208, in some embodiments, the cancer is a pancreatic cancer and the plurality of tissue types includes non-cancerous pancreatic tissue. However, this is not always true, particularly where the cancer is a metastatic cancer originating from a tissue distal from the tumor or tumors being imaged. Regardless, in some embodiments, it is advantageous for a physician to consider the proximity of organ systems to a tumor when evaluating how to treat a cancer and/or whether a tumor is operable. Accordingly, in some embodiments, a non-cancerous tissue, e.g., a nearby organ or part thereof, is displayed along with the tumor in a report described herein.

In yet other embodiments, the tumor being imaged is any solid tumor or tumors, e.g., a carcinoma, lymphoma, blastoma, glioblastoma, sarcoma, leukemia, breast cancer, squamous cell cancer, lung cancer, small-cell lung cancer, non-small cell lung cancer (NSCLC), adenocarcinoma of the lung, squamous carcinoma of the lung, head and neck cancer, cancer of the peritoneum, hepatocellular cancer, gastric or stomach cancer, pancreatic cancer, ovarian cancer, cervical cancer, liver cancer, bladder cancer, hepatoma, colon cancer, colorectal cancer, endometrial or uterine carcinoma, salivary gland carcinoma, kidney or renal cancer, liver cancer, prostate cancer, vulval cancer, thyroid cancer, hepatic carcinoma, B-cell lymphoma, low grade/follicular non-Hodgkin's lymphoma (NHL), small lymphocytic (SL) NHL, intermediate grade/follicular NHL, intermediate grade diffuse NHL, high grade immunoblastic NHL, high grade lymphoblastic NHL, high grade small non-cleaved cell NHL, bulky disease NHL, mantle cell lymphoma, AIDS-related lymphoma, Waldenstrom's Macroglobulinemia, chronic lymphocytic leukemia (CLL), acute lymphoblastic leukemia (ALL), hairy cell leukemia or chronic myeloblastic leukemia.

In some embodiments, the plurality of tissues includes one or more tissue from a supporting structure proximal to the tumor, such as arteries, veins, blood vessels, lymphatic vessels, and the like. For example, in some embodiments it is advantageous for a physician to consider the proximity of such structures when evaluating how to treat a cancer and/or whether a tumor is operable. Accordingly, in some embodiments, a nearby supporting structure is displayed along with the tumor in a report described herein. Accordingly, referring to block 210, in some embodiments, the plurality of tissue types includes lymph tissue. Similarly, referring to block 212, in some embodiments, the plurality of tissue types includes vascular tissue.

Referring to block 214, in some embodiments, method 200 includes segmenting the set of medical images by assigning, for each respective set of one or more pixels in a first plurality of sets of one or more pixels in the set of medical images, a label corresponding to a respective tissue type in the plurality of tissue types based on one or more corresponding pixel values for the respective set of one or more pixels. In some embodiments, the medical images are pre-segmented, and the method includes retrieving a result of the pre-segmentation, e.g., a tissue mask for the tumor and optionally a tissue mask for one or more other tissues identified in the imaging dataset.

Each pixel in a medical imaging dataset having multiple images corresponds to a unique position in a three-dimensional grid corresponding to the volume of the subject that was imaged. Accordingly, in some embodiments, each pixel is assigned a position in three-dimensional space. In some embodiments, the set of medical images is be segmented by evaluating features of the medical images, e.g., raw image intensity, filtered image intensity, and/or arithmetic combinations of filtered and/or unfiltered intensities, on a pixel by pixel basis.

Given the size of medical imaging sets, such as CT imaging sets, which commonly have a million or more pixels for each of hundreds of images, evaluating the data set on a pixel-by-pixel basis is time consuming. Moreover, this strategy is susceptible to pixel-to-pixel variations common in medical imaging. In some embodiments, to combat these issues, a three-dimensional voxel grid is established, where each respective voxel in a plurality of voxels in the voxel grid represents a plurality of pixels defining a volume in the three-dimensional space represented in the imaging dataset. For example, each voxel in the grid may consist of a 2-pixel x 2-pixel cube, a 3-pixel by 3-pixel cube, a 4-pixel x 4-pixel cube, a 5-pixel by 5-pixel cube, or larger cube. Use of voxels reduces the time and computational burden of segmenting the medical image set and accounts for pixel-to-pixel variation. In some embodiments, a feature value for a voxel is a measure of central tendency for the feature values of each pixel in the voxel.

In some embodiments, the three-dimensional voxel grid has at least 100 voxels. In some embodiments, the three-dimensional voxel grid has at least 1000 voxels. In some embodiments, the three-dimensional voxel grid has at least 10,000 voxels. In some embodiments, the three-dimensional voxel grid has at least 100,000 voxels. In some embodiments, the three-dimensional voxel grid has at least 1 million voxels. In some embodiments, the three-dimensional voxel grid has at least 10 million voxels. In some embodiments, the three-dimensional voxel grid has at least 100 million voxels. In some embodiments, the three-dimensional voxel grid has no more than 100 billion voxels. In some embodiments, the three-dimensional voxel grid has no more than 10 billion voxels. In some embodiments, the three-dimensional voxel grid has no more than 1 billion voxels. In some embodiments, the three-dimensional voxel grid has no more than 100 million voxels. In some embodiments, the three-dimensional voxel grid has no more than 10 million voxels.

In some embodiments, the three-dimensional voxel grid has from 100 voxels to 100 billion voxels. In some embodiments, the three-dimensional voxel grid has from 1000 voxels to 100 billion voxels. In some embodiments, the three-dimensional voxel grid has from 10,000 voxels to 100 billion voxels. In some embodiments, the three-dimensional voxel grid has from 100,000 voxels to 100 billion voxels. In some embodiments, the three-dimensional voxel grid has from 1 million voxels to 100 billion voxels. In some embodiments, the three-dimensional voxel grid has from 10 million voxels to 100 billion voxels. In some embodiments, the three-dimensional voxel grid has from 100 million voxels to 100 billion voxels. In some embodiments, the three-dimensional voxel grid has from 1 billion voxels to 100 billion voxels. In some embodiments, the three-dimensional voxel grid has from 100 voxels to 10 billion voxels. In some embodiments, the three-dimensional voxel grid has from 1000 voxels to 10 billion voxels. In some embodiments, the three-dimensional voxel grid has from 10,000 voxels to 10 billion voxels. In some embodiments, the three-dimensional voxel grid has from 100,000 voxels to 10 billion voxels. In some embodiments, the three-dimensional voxel grid has from 1 million voxels to 10 billion voxels. In some embodiments, the three-dimensional voxel grid has from 10 million voxels to 10 billion voxels. In some embodiments, the three-dimensional voxel grid has from 100 million voxels to 10 billion voxels. In some embodiments, the three-dimensional voxel grid has from 1 billion voxels to 10 billion voxels. In some embodiments, the three-dimensional voxel grid has from 100 voxels to 1 billion voxels. In some embodiments, the three-dimensional voxel grid has from 1000 voxels to 1 billion voxels. In some embodiments, the three-dimensional voxel grid has from 10,000 voxels to 1 billion voxels. In some embodiments, the three-dimensional voxel grid has from 100,000 voxels to 1 billion voxels. In some embodiments, the three-dimensional voxel grid has from 1 million voxels to 1 billion voxels. In some embodiments, the three-dimensional voxel grid has from 10 million voxels to 1 billion voxels. In some embodiments, the three-dimensional voxel grid has from 100 million voxels to 1 billion voxels. In some embodiments, the three-dimensional voxel grid has from 100 voxels to 100 million voxels. In some embodiments, the three-dimensional voxel grid has from 1000 voxels to 100 million voxels. In some embodiments, the three-dimensional voxel grid has from 10,000 voxels to 100 million voxels. In some embodiments, the three-dimensional voxel grid has from 100,000 voxels to 100 million voxels. In some embodiments, the three-dimensional voxel grid has from 1 million voxels to 100 million voxels. In some embodiments, the three-dimensional voxel grid has from 10 million voxels to 100 million voxels.

Examples of radiologic features useful for segmenting the image include, without limitation, shape features, first order features, gray level cooccurrence matrix (GLCM) features, gray level run length matrix (GLRLM) features, gray level size zone matrix (GLSZM), gray level dependence matrix (GLDM) features, and neighboring gray tone difference matrix (NGTDM) features.

Examples of shape features useful for segmenting medical images are listed in Table 1. Examples of first order features useful for segmenting medical images are listed in Table 2. Examples of GLCM features useful for segmenting medical images are listed in Table 3. Examples of GLRLM features useful for segmenting medical images are listed in Table 4. Examples of GLSZM features useful for segmenting medical images are listed in Table 5. Examples of GLDM features useful for segmenting medical images are listed in Table 6. Examples of NGTDM features useful for segmenting medical images are listed in Table 7.

**Table 1. Example shape features.**

| **Example Shape Features** |
|---|
| a mesh volume feature |
| a voxel volume feature |
| a surface area feature |
| a surface area to volume ratio feature |
| a three-dimensional (3D) sphericity feature |
| a first compactness feature |
| a second compactness feature |
| a 3D spherical disproportion feature |
| a maximum 3D diameter feature |
| a first 3D maximum two-dimensional (2D) diameter (*e.g*., slice) feature |
| a second 3D maximum 2D diameter (*e.g*., column) feature |
| a third 3D maximum 2D diameter (*e.g*., row) feature |
| a 3D major axis length feature |
| a 3D minor axis length feature |
| a least axis length feature |
| a 3D elongation feature |
| a flatness feature |
| a mesh surface feature |
| a pixel surface feature |
| a perimeter feature |
| a perimeter to surface ratio feature |
| a 2D sphericity feature |
| a 2D spherical disproportion feature |
| a first 2D maximum 2D diameter feature |
| a 2D major axis length feature |
| a 2D minor axis length feature |
| a 2D elongation feature |
| a roundness feature |
| an eccentricity feature |
| an asphericity feature |
| a center of mass shift feature |
| a first volume density (*e.g.,* axis-aligned bounding box) feature |
| a second volume density (*e.g.,* oriented minimum bounding box) feature |
| a third volume density (*e.g.,* approximate enclosing ellipsoid) feature |
| a fourth volume density (*e.g.,* minimum volume enclosing ellipsoid) feature |
| a fifth volume density (*e.g.,* convex hull) feature |
| a first area density (*e.g.,* axis-aligned bounding box) feature |
| a second area density (*e.g.,* oriented minimum bounding box) feature |
| a third area density (*e.g.,* approximate enclosing ellipsoid) feature |
| a fourth area density (*e.g.,* minimum volume enclosing ellipsoid) feature |
| a fifth area density (*e.g.,* convex hull) feature |
| an integrated intensity feature |
| a Moran's I index feature |
| a Geary's C measure feature |

**Table 2. Example first order features.**

| **Example First Order Features** |
|---|
| an energy feature |
| a total energy feature |
| an entropy feature |
| a minimum feature |
| a 10^{th} percentile feature |
| a 90^{th} percentile feature |
| a maximum feature |
| a mean feature |
| a median feature |
| an interquartile range feature |
| a range feature |
| a mean absolute deviation feature |
| a robust mean absolute deviation feature |
| a median absolute deviation feature |
| a root mean square feature |
| a standard deviation feature |
| a skewness feature |
| a variance feature |
| a kurtosis feature |
| a bin frequency feature |
| a local binary pattern (*e.g*., radius neighborhood) feature |
| a local peak feature |
| a global peak feature |
| a coefficient of variation feature |
| a quartile coefficient of dispersion feature |

**Table 3. Example GLCM features.**

| **Example GLCM Features** |
|---|
| an autocorrelation feature |
| a joint average feature |
| a cluster prominence feature |
| a cluster shade feature |
| a cluster tendency feature |
| a contrast (*e.g*., inertia) feature |
| a correlation feature |
| a difference average feature |
| a difference entropy feature |
| a difference variance feature |
| a joint energy feature |
| a joint entropy feature |
| a first informational measure of correlation (IMC1) feature |
| a second informational measure of correlation (IMC2) feature |
| an inverse difference moment (*e.g.,* homogeneity) feature |
| a maximal correlation coefficient feature |
| an inverse difference moment normalized feature |
| an inverse difference feature |
| an inverse difference normalized feature |
| an inverse variance feature |
| a maximum probability feature |
| a sum average feature |
| a sum entropy feature |
| a sum of squares feature |
| an energy (*e.g.,* angular second moment) feature |
| a variance feature |
| a joint variance feature |
| a joint maximum feature |
| a sum variance feature |
| a dissimilarity feature |

**Table 4. Example GLSZM features, where the terms "zone" and "area" are used interchangeably.**

| **Example GLSZM Features** |
|---|
| a small area emphasis feature |
| a large area emphasis feature |
| a gray level non-uniformity (GLN) feature |
| a GLN normalized feature |
| a size-zone non-uniformity (SZN) feature |
| a SZN normalized feature |
| a zone percentage feature |
| a gray level variance feature |
| a zone variance (*e.g.*, zone size variance) feature |
| a zone entropy (*e.g.,* zone size entropy) feature |
| a low gray level zone emphasis feature |
| a high gray level zone emphasis feature |
| a small area low gray level emphasis feature |
| a small area high gray level emphasis feature |
| a large area low gray level emphasis feature |
| a large area high gray level emphasis feature |

**Table 5. Example GLRLM features.**

| **Example GLRLM Features** |
|---|
| a short run emphasis (SRE) feature |
| a long run emphasis (LRE) feature |
| a gray level non-uniformity (GLN) feature |
| a GLN normalized (GLNN) feature |
| a run length non-uniformity (RLN) feature |
| a RLN normalized (RLNN) feature |
| a run percentage (RP) feature |
| a gray level variance (GLV) feature |
| a run variance (*e.g.,* run length variance) feature |
| a run entropy feature |
| a low gray level run emphasis (LGLRE) feature |
| a high gray level run emphasis (HGLRE) feature |
| a short run low gray level emphasis (SRLGLE) feature |
| a short run high gray level emphasis (SRHGLE) feature |
| a long run low gray level emphasis (LRLGLE) feature |
| a long run high gray level emphasis (LRHGLE) feature |

**Table 6. Example GLDM features.**

| **Example GLDM Features** |
|---|
| a small dependence emphasis feature |
| a large dependence emphasis feature |
| a gray level non-uniformity (GLN) feature |
| a dependence non-uniformity (DN) feature |
| a DN normalized (DNN) feature |
| a gray level variance feature |
| a dependence variance feature |
| a dependence entropy feature |
| a low gray level emphasis feature |
| a high gray level emphasis feature |
| a small dependence low gray level emphasis feature |
| a small dependence high gray level emphasis feature |
| a large dependence low gray level emphasis feature |
| a large dependence high gray level emphasis feature |

**Table 7. Example NGTDM features.**

| **Example NGTDM Features** |
|---|
| a coarseness feature |
| a contrast feature |
| a busyness feature |
| a complexity feature |
| a strength feature |

Methods for segmenting medical imaging data sets are known in the art. For example, conventionally, tissues and organ structures present in a medical image were segmented using signal thresholding techniques, e.g., that rely on differences between the densities of tissues to tell them apart. See, for example, Hu S., et al., "Automatic lung segmentation for accurate quantitation of volumetric X-ray CT images," IEEE Trans. Med. Imaging, 20(6):490-98 (2001), the disclosure of which is incorporated herein by reference. More recently, machine learning models have been used to improve and/or replace conventional thresholding tissue segmentation methodologies. For example, Skourt B.A., et al., "Lung CT Image Segmentation Using Deep Neural Networks," Procedia Computer Science, 127:109-13 (2018), the disclosure of which is incorporated herein by reference, describes use of a U-Net deep learning model to segment tissue in CT imaging data. Other examples of the use of machine learning models for tissue segmentation are described in M. Havaei et al., "Brain tumor segmentation with Deep Neural Networks," Med. Image Anal., 35:18-31 (2017); Akkus Z. et al., "Deep Learning for Brain MRI Segmentation: State of the Art and Future Directions," Journal of Digital Imaging, 30(4):449-59 (2017); and Badrinarayanan V., et al., "SegNet: A Deep Convolutional Encoder-Decoder Architecture for Image Segmentation," arXiv1511.00561 (2015), the disclosure of which are hereby incorporated by reference herein. Similarly, U.S. Patent No. 10,991,097, the disclosure of which is incorporated herein by reference, describes machine learning models for identifying cell types present in a slide. Similar methods for identifying cell types in medical imaging can be applied.

Non-limiting examples of models that can be used for image segmentation include a neural network, a support vector machine, a Naive Bayes model, a nearest neighbor model, a boosted trees model, a random forests model, and a clustering model. In some embodiments, a model is a machine learning model or algorithm. In some embodiments, a model is an unsupervised learning algorithm. One example of an unsupervised learning algorithm is cluster analysis.

In some embodiments, a model is supervised machine learning. Nonlimiting examples of supervised learning algorithms include, but are not limited to, logistic regression, neural networks, support vector machines, Naive Bayes algorithms, nearest neighbor algorithms, random forest algorithms, decision tree algorithms, boosted trees algorithms, multinomial logistic regression algorithms, linear models, linear regression, GradientBoosting, mixture models, hidden Markov models, Gaussian NB algorithms, linear discriminant analysis, or any combinations thereof. In some embodiments, a model is a multinomial classifier algorithm. In some embodiments, a model is a 2-stage stochastic gradient descent (SGD) model. In some embodiments, a model is a deep neural network *(e.g.,* a deep-and-wide sample-level classifier).

In some embodiments, an untrained model (*e.g.,* "untrained classifier" and/or "untrained neural network") includes a machine learning model or algorithm, such as a classifier or a neural network, that has not been trained on a target dataset. In some embodiments, training a model (*e.g.,* training a neural network) refers to the process of training an untrained or partially trained model *(e.g.,* an untrained or partially trained neural network). For instance, consider the case of a plurality of training samples comprising a corresponding plurality of medical images (*e.g.,* of a medical dataset). The plurality of medical images is applied as collective input to an untrained or partially trained model, in conjunction with a corresponding measured indication of one or more features for each respective medical image (hereinafter training dataset) to train the untrained or partially trained model on indications that identify features related to morphological classes, thereby obtaining a trained model. Moreover, it will be appreciated that the term "untrained model" does not exclude the possibility that transfer learning techniques are used in such training of the untrained or partially trained model. For instance, Fernandes et al., 2017, "Transfer Learning with Partial Observability Applied to Cervical Cancer Screening," Pattern Recognition and Image Analysis: 8th Iberian Conference Proceedings, 243-250, which is hereby incorporated by reference in its entirety for all purposes, provides non-limiting examples of such transfer learning. In instances where transfer learning is used, the untrained model described above is provided with additional data over and beyond that of the primary training dataset. That is, in non-limiting examples of transfer learning embodiments, the untrained model receives (i) the plurality of images and the measured indications for each respective image ("primary training dataset") and (ii) additional data. In some embodiments, this additional data is in the form of parameters (*e.g.,* coefficients, weights, and/or hyperparameters) that were learned from another, auxiliary training dataset. Moreover, while a description of a single auxiliary training dataset has been disclosed, it will be appreciated that there is no limit on the number of auxiliary training datasets that may be used to complement the primary training dataset in training the untrained model in the present disclosure. For instance, in some embodiments, two or more auxiliary training datasets, three or more auxiliary training datasets, four or more auxiliary training datasets or five or more auxiliary training datasets are used to complement the primary training dataset through transfer learning, where each such auxiliary dataset is different than the primary training dataset. Any manner of transfer learning may be used in such embodiments. For instance, consider the case where there is a first auxiliary training dataset and a second auxiliary training dataset in addition to the primary training dataset. The parameters learned from the first auxiliary training dataset (by application of a first model to the first auxiliary training dataset) may be applied to the second auxiliary training dataset using transfer learning techniques (*e.g.,* a second model that is the same or different from the first model), which in turn may result in a trained intermediate model whose parameters are then applied to the primary training dataset and this, in conjunction with the primary training dataset itself, is applied to the untrained model. Alternatively, a first set of parameters learned from the first auxiliary training dataset (by application of a first model to the first auxiliary training dataset) and a second set of parameters learned from the second auxiliary training dataset (by application of a second model that is the same or different from the first model to the second auxiliary training dataset) may each individually be applied to a separate instance of the primary training dataset (*e.g.,* by separate independent matrix multiplications) and both such applications of the parameters to separate instances of the primary training dataset in conjunction with the primary training dataset itself (or some reduced form of the primary training dataset such as principal components or regression coefficients learned from the primary training set) may then be applied to the untrained model in order to train the untrained model. In some instances, additionally or alternatively, knowledge regarding objects related to morphological classes derived from an auxiliary training dataset is used, in conjunction with the object and/or class-labeled images in the primary training dataset, to train the untrained model.

*Neural network algorithms,* also known as artificial neural networks (ANNs), include convolutional and/or residual neural network algorithms (deep learning algorithms). Neural networks can be machine learning algorithms that may be trained to map an input data set to an output data set, where the neural network includes an interconnected group of nodes organized into multiple layers of nodes. For example, the neural network architecture may include at least an input layer, one or more hidden layers, and an output layer. The neural network may include any total number of layers, and any number of hidden layers, where the hidden layers function as trainable feature extractors that allow mapping of a set of input data to an output value or set of output values. As used herein, a deep learning algorithm (DNN) can be a neural network comprising a plurality of hidden layers, *e.g.,* two or more hidden layers. Each layer of the neural network can include a number of nodes (or "neurons"). A node can receive input that comes either directly from the input data or the output of nodes in previous layers, and perform a specific operation, *e.g.,* a summation operation. In some embodiments, a connection from an input to a node is associated with a parameter (*e.g.,* a weight and/or weighting factor). In some embodiments, the node may sum up the products of all pairs of inputs, xᵢ, and their associated parameters. In some embodiments, the weighted sum is offset with a bias, b. In some embodiments, the output of a node or neuron may be gated using a threshold or activation function, f, which may be a linear or non-linear function. The activation function may be, for example, a rectified linear unit (ReLU) activation function, a Leaky ReLU activation function, or other function such as a saturating hyperbolic tangent, identity, binary step, logistic, arcTan, softsign, parametric rectified linear unit, exponential linear unit, softPlus, bent identity, softExponential, Sinusoid, Sine, Gaussian, or sigmoid function, or any combination thereof.

The weighting factors, bias values, and threshold values, or other computational parameters of the neural network, may be "taught" or "learned" in a training phase using one or more sets of training data. For example, the parameters may be trained using the input data from a training data set and a gradient descent or backward propagation method so that the output value(s) that the ANN computes are consistent with the examples included in the training data set. The parameters may be obtained from a back propagation neural network training process.

Any of a variety of neural networks may be suitable for use in performing the methods disclosed herein. Examples can include, but are not limited to, feedforward neural networks, radial basis function networks, recurrent neural networks, residual neural networks, convolutional neural networks, residual convolutional neural networks, and the like, or any combination thereof. In some embodiments, the machine learning makes use of a pre-trained and/or transfer-learned ANN or deep learning architecture. Convolutional and/or residual neural networks can be used for analyzing an image of a subject in accordance with the present disclosure.

For instance, a deep neural network model includes an input layer, a plurality of individually parameterized (*e.g.,* weighted) convolutional layers, and an output scorer. The parameters (*e.g.,* weights) of each of the convolutional layers as well as the input layer contribute to the plurality of parameters (*e.g.,* weights) associated with the deep neural network model. In some embodiments, at least 100 parameters, at least 1000 parameters, at least 10,000 parameters, at least 100,000 parameters, at least 1 million parameters, at least 10 million parameters, or more parameters are associated with the deep neural network model. As such, deep neural network models require a computer to be used because they cannot be mentally solved. In other words, given an input to the model, the model output needs to be determined using a computer rather than mentally in such embodiments. *See,* for example, Krizhevsky et al., 2012, "Imagenet classification with deep convolutional neural networks," in Advances in Neural Information Processing Systems 2, Pereira, Burges, Bottou, Weinberger, eds., pp. 1097-1105, Curran Associates, Inc.; Zeiler, 2012 "ADADELTA: an adaptive learning rate method," CoRR, vol. abs/1212.5701; and Rumelhart et al., 1988, "Neurocomputing: Foundations of research," ch. Learning Representations by Back-propagating Errors, pp. 696-699, Cambridge, MA, USA: MIT Press, each of which is hereby incorporated by reference in its entirety for all purposes.

Neural network algorithms, including convolutional neural network algorithms, suitable for use as models are disclosed in, for example, Vincent et al., 2010, "Stacked denoising autoencoders: Learning useful representations in a deep network with a local denoising criterion," J Mach Learn Res 11, pp. 3371-3408; Larochelle et al., 2009, "Exploring strategies for training deep neural networks," J Mach Learn Res 10, pp. 1-40; and Hassoun, 1995, Fundamentals of Artificial Neural Networks, Massachusetts Institute of Technology, each of which is hereby incorporated by reference in its entirety for all purposes. Additional example neural networks suitable for use as models are disclosed in Duda et al., 2001, Pattern Classification, Second Edition, John Wiley & Sons, Inc., New York; and Hastie et al., 2001, The Elements of Statistical Learning, Springer-Verlag, New York, each of which is hereby incorporated by reference in its entirety for all purposes. Additional example neural networks suitable for use as models are also described in Draghici, 2003, Data Analysis Tools for DNA Microarrays, Chapman & Hall/CRC; and Mount, 2001, Bioinformatics: sequence and genome analysis, Cold Spring Harbor Laboratory Press, Cold Spring Harbor, New York, each of which is hereby incorporated by reference in its entirety for all purposes.

*Support vector machines.* In some embodiments, the model is a support vector machine (SVM). SVM algorithms suitable for use as models are described in, for example, Cristianini and Shawe-Taylor, 2000, "An Introduction to Support Vector Machines," Cambridge University Press, Cambridge; Boser et al., 1992, "A training algorithm for optimal margin classifiers," in Proceedings of the 5th Annual ACM Workshop on Computational Learning Theory, ACM Press, Pittsburgh, Pa., pp. 142-152; Vapnik, 1998, Statistical Learning Theory, Wiley, New York; Mount, 2001, Bioinformatics: sequence and genome analysis, Cold Spring Harbor Laboratory Press, Cold Spring Harbor, N.Y.; Duda, Pattern Classification, Second Edition, 2001, John Wiley & Sons, Inc., pp. 259, 262-265; and Hastie, 2001, The Elements of Statistical Learning, Springer, New York; and Furey et al., 2000, Bioinformatics 16, 906-914, each of which is hereby incorporated by reference in its entirety for all purposes. When used for classification, SVMs separate a given set of binary labeled data with a hyper-plane that is maximally distant from the labeled data. For cases in which no linear separation is possible, SVMs can work in combination with the technique of 'kernels', which automatically realizes a non-linear mapping to a feature space. The hyper-plane found by the SVM in feature space can correspond to a non-linear decision boundary in the input space. In some embodiments, the plurality of parameters (*e.g.,* weights) associated with the SVM define the hyper-plane. In some embodiments, the hyper-plane is defined by at least 10, at least 20, at least 50, or at least 100 parameters and the SVM model requires a computer to calculate because it cannot be mentally solved.

*Naive Bayes algorithms.* In some embodiments, the model is a Naive Bayes algorithm. Naive Bayes classifiers suitable for use as models are disclosed, for example, in Ng et al., 2002, "On discriminative vs. generative classifiers: A comparison of logistic regression and naive Bayes," Advances in Neural Information Processing Systems, 14, which is hereby incorporated by reference in its entirety for all purposes. A Naive Bayes classifier is any classifier in a family of "probabilistic classifiers" based on applying Bayes' theorem with strong (naive) independence assumptions between the features. In some embodiments, they are coupled with Kernel density estimation. *See,* for example, Hastie et al., 2001, The elements of statistical learning : data mining, inference, and prediction, eds. Tibshirani and Friedman, Springer, New York, which is hereby incorporated by reference in its entirety for all purposes.

*Nearest neighbor algorithms.* In some embodiments, a model is a nearest neighbor algorithm. Nearest neighbor models can be memory-based and include no model to be fit. For nearest neighbors, given a query point x₀ (a first image), the k training points x_{(*r*)}, r, ..., k (here the training images) closest in distance to x₀ are identified and then the point x₀ is classified using the k nearest neighbors. In some embodiments, the distance to these neighbors is a function of the values of a discriminating set. In some embodiments, Euclidean distance in feature space is used to determine distance as *d*_{(*i*)} = ∥*x*_{(*i*)} - *x*_{(*o*)}∥*.* In some embodiments, when the nearest neighbor algorithm is used, the value data used to compute the linear discriminant is standardized to have mean zero and variance 1. The nearest neighbor rule can be refined to address issues of unequal class priors, differential misclassification costs, and feature selection. Many of these refinements involve some form of weighted voting for the neighbors. For more information on nearest neighbor analysis, *see* Duda, Pattern Classification, Second Edition, 2001, John Wiley & Sons, Inc; and Hastie, 2001, The Elements of Statistical Learning, Springer, New York, each of which is hereby incorporated by reference in its entirety for all purposes.

A k-nearest neighbor model is a non-parametric machine learning method in which the input consists of the k closest training examples in feature space. The output is a class membership. An object is classified by a plurality vote of its neighbors, with the object being assigned to the class most common among its k nearest neighbors (k is a positive integer, typically small). If k = 1, then the object is simply assigned to the class of that single nearest neighbor. *See,* Duda et al., 2001, Pattern Classification, Second Edition, John Wiley & Sons, which is hereby incorporated by reference in its entirety for all purposes. In some embodiments, the number of distance calculations needed to solve the k-nearest neighbor model is such that a computer is used to solve the model for a given input because it cannot be mentally performed.

*Random forest*, *decision tree, and boosted tree algorithms.* In some embodiments, the model is a decision tree. Decision trees suitable for use as models are described generally by Duda, 2001, Pattern Classification, John Wiley & Sons, Inc., New York, pp. 395-396, which is hereby incorporated by reference in its entirety for all purposes. Tree-based methods partition the feature space into a set of rectangles, and then fit a model (like a constant) in each one. In some embodiments, the decision tree is random forest regression. One specific algorithm that can be used is a classification and regression tree (CART). Other specific decision tree algorithms include, but are not limited to, ID3, C4.5, MART, and Random Forests. CART, ID3, and C4.5 are described in Duda, 2001, Pattern Classification, John Wiley & Sons, Inc., New York, pp. 396-408 and pp. 411-412, which is hereby incorporated by reference in its entirety for all purposes. CART, MART, and C4.5 are described in Hastie et al., 2001, The Elements of Statistical Learning, Springer-Verlag, New York, Chapter 9, which is hereby incorporated by reference in its entirety for all purposes. Random Forests are described in Breiman, 1999, "Random Forests--Random Features," Technical Report 567, Statistics Department, U.C. Berkeley, September 1999, which is hereby incorporated by reference in its entirety for all purposes. In some embodiments, the decision tree model includes at least 10, at least 20, at least 50, or at least 100 parameters (*e.g.,* weights and/or decisions) and requires a computer to calculate because it cannot be mentally solved.

*Linear discriminant analysis algorithms.* Linear discriminant analysis (LDA), normal discriminant analysis (NDA), or discriminant function analysis can be a generalization of Fisher's linear discriminant, a method used in statistics, pattern recognition, and machine learning to find a linear combination of features that characterizes or separates two or more classes of objects or events. The resulting combination can be used as the model *(e.g.,* a linear classifier) in some embodiments of the present disclosure.

*Mixture model and Hidden Markov model.* In some embodiments, the model is a mixture model, such as that described in McLachlan et al., Bioinformatics 18(3):413-422, 2002. In some embodiments, in particular, those embodiments including a temporal component, the model is a hidden Markov model such as described by Schliep et al., 2003, Bioinformatics 19(1):i255-i263.

*Clustering.* In some embodiments, the model is an unsupervised clustering model. In some embodiments, the model is a supervised clustering model. Clustering algorithms suitable for use as models are described, for example, at pages 211-256 of Duda and Hart, Pattern Classification and Scene Analysis, 1973, John Wiley & Sons, Inc., New York, (hereinafter "Duda 1973") which is hereby incorporated by reference in its entirety for all purposes. The clustering problem can be described as one of finding natural groupings in a dataset. To identify natural groupings, two issues can be addressed. First, a way to measure similarity (or dissimilarity) between two samples can be determined. This metric (*e.g.,* similarity measure) can be used to ensure that the samples in one cluster are more like one another than they are to samples in other clusters. Second, a mechanism for partitioning the data into clusters using the similarity measure can be determined. One way to begin a clustering investigation can be to define a distance function and to compute the matrix of distances between all pairs of samples in a training dataset. If distance is a good measure of similarity, then the distance between reference entities in the same cluster can be significantly less than the distance between the reference entities in different clusters. However, clustering may not use a distance metric. For example, a nonmetric similarity function s(x, x') can be used to compare two vectors x and x'. s(x, x') can be a symmetric function whose value is large when x and x' are somehow "similar." Once a method for measuring "similarity" or "dissimilarity" between points in a dataset has been selected, clustering can use a criterion function that measures the clustering quality of any partition of the data. Partitions of the data set that extremize the criterion function can be used to cluster the data. Particular exemplary clustering techniques that can be used in the present disclosure can include, but are not limited to, hierarchical clustering (agglomerative clustering using a nearest-neighbor algorithm, farthest-neighbor algorithm, the average linkage algorithm, the centroid algorithm, or the sum-of-squares algorithm), k-means clustering, fuzzy k-means clustering algorithm, and Jarvis-Patrick clustering. In some embodiments, the clustering includes unsupervised clustering (*e.g.,* with no preconceived number of clusters and/or no predetermination of cluster assignments).

*Ensembles of models and boosting.* In some embodiments, an ensemble (two or more) of models is used. In some embodiments, a boosting technique such as AdaBoost is used in conjunction with many other types of learning algorithms to improve the performance of the model. In this approach, the output of any of the models disclosed herein, or their equivalents, is combined into a weighted sum that represents the final output of the boosted model. In some embodiments, the plurality of outputs from the models is combined using any measure of central tendency known in the art, including but not limited to a mean, median, mode, a weighted mean, weighted median, weighted mode, *etc.* In some embodiments, the plurality of outputs is combined using a voting method. In some embodiments, a respective model in the ensemble of models is weighted or unweighted.

One of skill in the art will readily appreciate other models that are applicable to the systems and methods of the present disclosure. In some embodiments, the systems, methods, and devices of the present disclosure utilize more than one model to provide an evaluation (*e.g.,* arrive at an evaluation given one or more inputs) with an increased accuracy. For instance, in some embodiments, each respective model arrives at a corresponding evaluation when provided a respective data set. Accordingly, each respective model can independently arrive at a result and then the result of each respective model is collectively verified through a comparison or amalgamation of the models. From this, a cumulative result is provided by the models. However, the present disclosure is not limited thereto.

Referring to block 216, in some embodiments, method 200 includes generating, for each respective tissue type in the plurality of tissue types, a corresponding mask based on respective sets of one or more pixels assigned the label corresponding to the respective tissue type. In some embodiments, a corresponding mask is a binary construct having, for each position in a three dimensional grid representing a volume of the subject, either a first value (e.g., a "1") indicating that the tissue is of the tissue type represented by the mask or a second value (e.g., a "0") indicating that the tissue is not of the tissue type represented by the mask.

Referring to block 217, in some embodiments, method 200 includes generating a visual representation of the tumor tissue of the subject based on the corresponding mask for the tumor tissue. In some embodiments, referring to block 218, this includes generating a corresponding mesh surface for the tumor tissue based on an outer boundary of the corresponding mask for the tumor tissue and smoothing edges of the corresponding mesh surface for the tumor tissue. For example, Figure 4 illustrates visualization of smoothed masks for a pancreatic tumor 402 and pancreas 404.

Referring to block 220, in some embodiments, each respective set of one or more pixels in the first plurality of sets of one or more pixels corresponds to a respective voxel in a uniform three-dimensional grid of voxels defined for the set of medical images and for a respective tissue type in the plurality of tissue types, the corresponding mask comprises a binary indication, for each respective voxel in the three-dimensional grid of voxels, of whether the tissue represented in the voxel is the respective tissue type.

In some embodiments, the three-dimensional grid is the same as a three-dimensional voxel grid established for segmenting the medical images. In other embodiments, the three dimensional grid represents a resampling of the input volumes to a regular spacing, e.g., a spacing representative of the spacing of the tissue in the subject. An example method for resampling medical imaging data is described in Li A., et al., "Methods for efficient, high quality volume resampling in the frequency domain," IEEE Visualization, Austin, TX, USA, pp. 3-10, (2004) doi: 10.1109/VISUAL.2004.70, the disclosure of which is incorporated herein by reference.

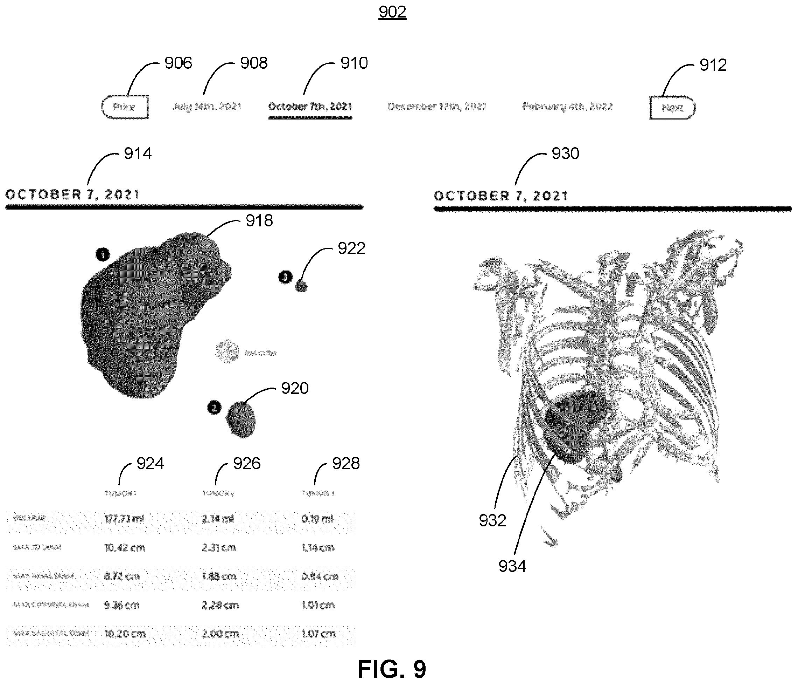

Referring to block 222, in some embodiments, the corresponding mask for the tumor tissue comprises a plurality of groups of non-zero voxels, wherein each respective group of non-zero voxels in the plurality of groups of non-zero voxels is separated from every other respective group of non-zero voxels in the plurality of groups of non-zero voxels by at least one zero voxel. For example, many cancers manifest as multiple tumor islands, separated in space within the body. For example, panel 914 in radiology report 902 provides a visual representation of a pancreatic cancer with three tumor islands 918, 920, and 922, as illustrated in Figure 9.

Referring to block 224, in some embodiments, generating the visual representation of the tumor tissue comprises generating a corresponding mesh surface for each respective group of non-zero voxels in the plurality of groups of non-zero voxels. The is, in some embodiments, the system identifies each tumor island represented in the tumor tissue mask and generates a separate mask for each island, e.g., again as illustrated in panel 914 in radiology report 902 illustrated in Figure 9.

Referring to block 226, in some embodiments, the visual representation of the tumor tissue excludes representations of respective groups of non-zero voxels having a total volume below a first volume threshold. That is, in some embodiments, the system does not display tumor islands below a threshold volume. For example, panel 914 in radiology report 902 provides a visual representation of a pancreatic cancer with three tumor islands 918, 920, and 922, as illustrated in Figure 9. Tumor island 922 has the smallest total volume of 0.19 mL. However, smaller tumor islands represented in the mask for the tumor tissue may not be displayed in this panel because they have a volume that is smaller than a threshold volume, e.g., 0.01 mL, 0.05 mL, 0.1 mL, etc. In some embodiments, this simplifies the image provided to the physician while reporting all substantial tumor masses. In some embodiments, this accounts for background noise in the segmentation process that may result in the false identification of small tumor islands in the data.

Referring to block 228, in some embodiments, method 200 includes determining a volume for the cancer in the subject based on a volume contained within the mesh surface for the tumor tissue. Referring to block 230, in some embodiments, the volume is determined after smoothing edges of the corresponding mesh surface for the tumor tissue.

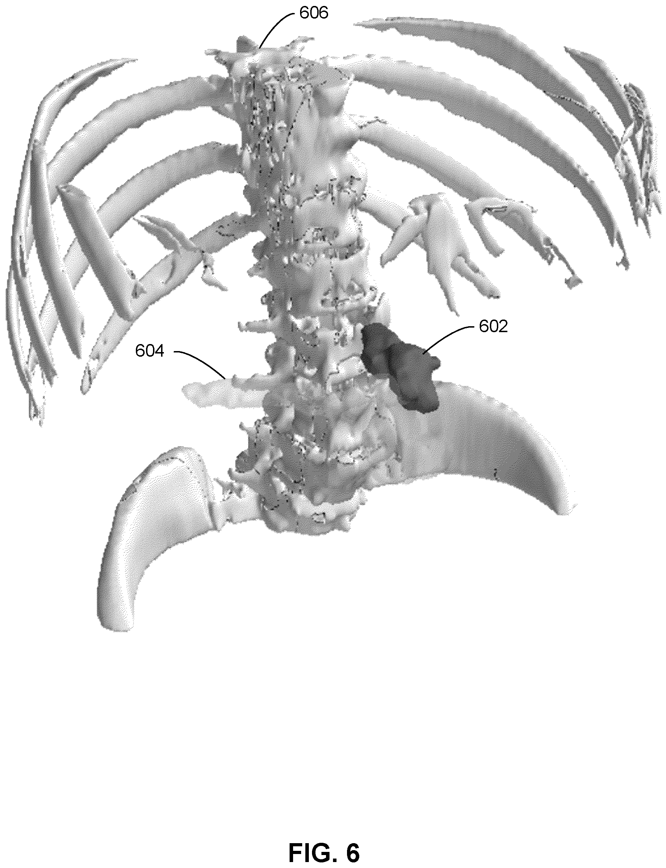

Referring to block 231, in some embodiments, method 200 includes generating a visual representation of a skeleton of the subject within the region of interest based on the first set of medical images. For example, Figure 6 illustrates a visual representation of a pancreatic tumor 602 and pancreas 604 positioned relative to a visual representation of the skeleton 606 of the patient. Visualization of the skeleton of the subject provides the physician with spatial context for the tumor, informing treatment and/or surgical intervention decisions.

Referring to block 232, in some embodiments, generating the visual representation of the skeleton comprises determining, for each respective set of one or more pixels in a second plurality of sets of one or more pixels in the set of medical images, a corresponding tissue density. In some embodiments, the second plurality of sets of one or more pixels is a three-dimensional voxel grid established for segmenting tissues within the medical images. In other embodiments, the second plurality of sets of one or more pixels is a three-dimensional voxel grid is different than a three-dimensional voxel grid established for segmenting tissues within the medical images.

Referring to block 234, in some embodiments, method 200 includes generating a corresponding mask for the skeleton based on respective sets of one or more pixels in the second plurality of sets of one or more pixels having a tissue density satisfying a set of one or more bone density criteria. That is, in some embodiments, bone is identified in the set of medical images by simply thresholding an intensity value for the images, rather than through segmentation as done for the tumor tissue and one or more non-cancerous tissues. This is because healthy bones have a substantially greater density than do other tissues. An example schema for thresholding intensity for distinguishing soft tissue from bone is provided in Chougule V.N., et al., "Clinical Case Study: Spine Modeling for Minimum Invasive Spine Surgeries (MISS) using Rapid Prototyping," Procedia Engineering 97:212-19 (2014), the disclosure of which is incorporated herein by reference.

Where the second plurality of sets of one or more pixels is a three-dimensional voxel grid is different than a three-dimensional voxel grid established for segmenting tissues within the medical images, one or more of the resulting tissue masks is resampled to ensure that all tissues being imaged are set in the same coordinate system.

Referring to block 236, in some embodiments, the first set of medical images comprises a computed tomography (CT) scan and the set of one or more bone density criteria comprise a first criterion that is satisfied by a measure of central tendency for the pixel intensity of the respective set of one or more pixels of greater than a first threshold of from 300 to 500 Hounsfield units (HUs). In some embodiments, the first threshold is from 200 to 600 HU. In some embodiments, the first threshold is from 200 to 550 HU. In some embodiments, the first threshold is from 200 to 500 HU. In some embodiments, the first threshold is from 200 to 475 HU. In some embodiments, the first threshold is from 200 to 450 HU. In some embodiments, the first threshold is from 200 to 425 HU. In some embodiments, the first threshold is from 200 to 400 HU. In some embodiments, the first threshold is from 250 to 600 HU. In some embodiments, the first threshold is from 250 to 550 HU. In some embodiments, the first threshold is from 250 to 500 HU. In some embodiments, the first threshold is from 250 to 475 HU. In some embodiments, the first threshold is from 250 to 450 HU. In some embodiments, the first threshold is from 250 to 425 HU. In some embodiments, the first threshold is from 250 to 400 HU. In some embodiments, the first threshold is from 275 to 600 HU. In some embodiments, the first threshold is from 275 to 550 HU. In some embodiments, the first threshold is from 275 to 500 HU. In some embodiments, the first threshold is from 275 to 475 HU. In some embodiments, the first threshold is from 275 to 450 HU. In some embodiments, the first threshold is from 275 to 425 HU. In some embodiments, the first threshold is from 275 to 400 HU. In some embodiments, the first threshold is from 300 to 600 HU. In some embodiments, the first threshold is from 300 to 550 HU. In some embodiments, the first threshold is from 300 to 500 HU. In some embodiments, the first threshold is from 300 to 475 HU. In some embodiments, the first threshold is from 300 to 450 HU. In some embodiments, the first threshold is from 300 to 425 HU. In some embodiments, the first threshold is from 300 to 400 HU. In some embodiments, the first threshold is from 325 to 600 HU. In some embodiments, the first threshold is from 325 to 550 HU. In some embodiments, the first threshold is from 325 to 500 HU. In some embodiments, the first threshold is from 325 to 475 HU. In some embodiments, the first threshold is from 325 to 450 HU. In some embodiments, the first threshold is from 325 to 425 HU. In some embodiments, the first threshold is from 325 to 400 HU. In some embodiments, the first threshold is from 350 to 600 HU. In some embodiments, the first threshold is from 350 to 550 HU. In some embodiments, the first threshold is from 350 to 500 HU. In some embodiments, the first threshold is from 350 to 475 HU. In some embodiments, the first threshold is from 350 to 450 HU. In some embodiments, the first threshold is from 350 to 425 HU. In some embodiments, the first threshold is from 350 to 400 HU. In some embodiments, the first threshold is 200, 225, 250, 275, 300, 325, 350, 375, 400, 425, 450, 475, 500, 525, 550, 575, or 600 HU. In some embodiments, the first threshold is 200±5%, 225±5%, 250±5%, 275±5%, 300±5%, 325±5%, 350±5%, 375±5%, 400±5%, 425±5%, 450±5%, 475±5%, 500±5%, 525±5%, 550±5%, 575±5%, or 600±5% HU. In some embodiments, the first threshold is 200±10%, 225±10%, 250±10%, 275±10%, 300±10%, 325±10%, 350±10%, 375±10%, 400±10%, 425±10%, 450±10%, 475±10%, 500±10%, 525±10%, 550±10%, 575±10%, or 600±10% HU.

Referring to block 238, in some embodiments, the first set of medical images comprises a computed tomography (CT) scan and the set of one or more bone density criteria comprise a second criterion that is satisfied by a measure of central tendency for the pixel intensity of the respective set of one or more pixels of less than greater than a second threshold of from 1750 to 2500 Hounsfield units (HUs). In some embodiments, the second threshold is from 1600 to 3000 HU. In some embodiments, the second threshold is from 1600 to 2900 HU. In some embodiments, the second threshold is from 1600 to 2800 HU. In some embodiments, the second threshold is from 1600 to 27000 HU. In some embodiments, the second threshold is from 1600 to 2600 HU. In some embodiments, the second threshold is from 1600 to 2550 HU. In some embodiments, the second threshold is from 1600 to 2500 HU. In some embodiments, the second threshold is from 1600 to 2450 HU. In some embodiments, the second threshold is from 1600 to 2400 HU. In some embodiments, the second threshold is from 1600 to 2350 HU. In some embodiments, the second threshold is from 1600 to 2300 HU. In some embodiments, the second threshold is from 1650 to 3000 HU. In some embodiments, the second threshold is from 1650 to 2900 HU. In some embodiments, the second threshold is from 1650 to 2800 HU. In some embodiments, the second threshold is from 1650 to 27000 HU. In some embodiments, the second threshold is from 1650 to 2600 HU. In some embodiments, the second threshold is from 1650 to 2550 HU. In some embodiments, the second threshold is from 1650 to 2500 HU. In some embodiments, the second threshold is from 1650 to 2450 HU. In some embodiments, the second threshold is from 1650 to 2400 HU. In some embodiments, the second threshold is from 1650 to 2350 HU. In some embodiments, the second threshold is from 1650 to 2300 HU. In some embodiments, the second threshold is from 1700 to 3000 HU. In some embodiments, the second threshold is from 1700 to 2900 HU. In some embodiments, the second threshold is from 1700 to 2800 HU. In some embodiments, the second threshold is from 1700 to 27000 HU. In some embodiments, the second threshold is from 1700 to 2600 HU. In some embodiments, the second threshold is from 1700 to 2550 HU. In some embodiments, the second threshold is from 1700 to 2500 HU. In some embodiments, the second threshold is from 1700 to 2450 HU. In some embodiments, the second threshold is from 1700 to 2400 HU. In some embodiments, the second threshold is from 1700 to 2350 HU. In some embodiments, the second threshold is from 1700 to 2300 HU. In some embodiments, the second threshold is from 1725 to 3000 HU. In some embodiments, the second threshold is from 1725 to 2900 HU. In some embodiments, the second threshold is from 1725 to 2800 HU. In some embodiments, the second threshold is from 1725 to 27000 HU. In some embodiments, the second threshold is from 1725 to 2600 HU. In some embodiments, the second threshold is from 1725 to 2550 HU. In some embodiments, the second threshold is from 1725 to 2500 HU. In some embodiments, the second threshold is from 1725 to 2450 HU. In some embodiments, the second threshold is from 1725 to 2400 HU. In some embodiments, the second threshold is from 1725 to 2350 HU. In some embodiments, the second threshold is from 1725 to 2300 HU. In some embodiments, the second threshold is from 1750 to 3000 HU. In some embodiments, the second threshold is from 1750 to 2900 HU. In some embodiments, the second threshold is from 1750 to 2800 HU. In some embodiments, the second threshold is from 1750 to 27000 HU. In some embodiments, the second threshold is from 1750 to 2600 HU. In some embodiments, the second threshold is from 1750 to 2550 HU. In some embodiments, the second threshold is from 1750 to 2500 HU. In some embodiments, the second threshold is from 1750 to 2450 HU. In some embodiments, the second threshold is from 1750 to 2400 HU. In some embodiments, the second threshold is from 1750 to 2350 HU. In some embodiments, the second threshold is from 1750 to 2300 HU. In some embodiments, the second threshold is from 1775 to 3000 HU. In some embodiments, the second threshold is from 1775 to 2900 HU. In some embodiments, the second threshold is from 1775 to 2800 HU. In some embodiments, the second threshold is from 1775 to 27000 HU. In some embodiments, the second threshold is from 1775 to 2600 HU. In some embodiments, the second threshold is from 1775 to 2550 HU. In some embodiments, the second threshold is from 1775 to 2500 HU. In some embodiments, the second threshold is from 1775 to 2450 HU. In some embodiments, the second threshold is from 1775 to 2400 HU. In some embodiments, the second threshold is from 1775 to 2350 HU. In some embodiments, the second threshold is from 1775 to 2300 HU. In some embodiments, the second threshold is from 1800 to 3000 HU. In some embodiments, the second threshold is from 1800 to 2900 HU. In some embodiments, the second threshold is from 1800 to 2800 HU. In some embodiments, the second threshold is from 1800 to 27000 HU. In some embodiments, the second threshold is from 1800 to 2600 HU. In some embodiments, the second threshold is from 1800 to 2550 HU. In some embodiments, the second threshold is from 1800 to 2500 HU. In some embodiments, the second threshold is from 1800 to 2450 HU. In some embodiments, the second threshold is from 1800 to 2400 HU. In some embodiments, the second threshold is from 1800 to 2350 HU. In some embodiments, the second threshold is from 1800 to 2300 HU. In some embodiments, the second threshold is from 1850 to 3000 HU. In some embodiments, the second threshold is from 1850 to 2900 HU. In some embodiments, the second threshold is from 1850 to 2800 HU. In some embodiments, the second threshold is from 1850 to 27000 HU. In some embodiments, the second threshold is from 1850 to 2600 HU. In some embodiments, the second threshold is from 1850 to 2550 HU. In some embodiments, the second threshold is from 1850 to 2500 HU. In some embodiments, the second threshold is from 1850 to 2450 HU. In some embodiments, the second threshold is from 1850 to 2400 HU. In some embodiments, the second threshold is from 1850 to 2350 HU. In some embodiments, the second threshold is from 1850 to 2300 HU. In some embodiments, the second threshold is from 1900 to 3000 HU. In some embodiments, the second threshold is from 1900 to 2900 HU. In some embodiments, the second threshold is from 1900 to 2800 HU. In some embodiments, the second threshold is from 1900 to 27000 HU. In some embodiments, the second threshold is from 1900 to 2600 HU. In some embodiments, the second threshold is from 1900 to 2550 HU. In some embodiments, the second threshold is from 1900 to 2500 HU. In some embodiments, the second threshold is from 1900 to 2450 HU. In some embodiments, the second threshold is from 1900 to 2400 HU. In some embodiments, the second threshold is from 1900 to 2350 HU. In some embodiments, the second threshold is from 1900 to 2300 HU. In some embodiments, the second threshold is 1600, 1625, 1650, 1675, 1700, 1725, 1750, 1775, 1800, 1825, 1850, 1875, or 1900 HU. In some embodiments, the second threshold is 1600±5%, 1625±5%, 1650±5%, 1675±5%, 1700±5%, 1725±5%, 1750±5%, 1775±5%, 1800±5%, 1825±5%, 1850±5%, 1875±5%, or 1900±5% HU. In some embodiments, the second threshold is 1600±10%, 1625±10%, 1650±10%, 1675±10%, 1700±10%, 1725±10%, 1750±10%, 1775±10%, 1800±10%, 1825±10%, 1850±10%, 1875±10%, or 1900±10% HU.

Referring to block 240, in some embodiments, the method then includes generating a corresponding mesh surface for the skeleton based on an outer boundary of the corresponding mask for the skeleton. Referring to block 242, in some embodiments, the method includes smoothing the edges of the corresponding mesh surface for the skeleton.

Referring to block 244, in some embodiments, each respective set of one or more pixels in the second plurality of sets of one or more pixels corresponds to a respective voxel in a second three-dimensional grid of voxels defined for the set of medical images and the corresponding mask for the skeleton comprises a binary indication, for each respective voxel in the second three-dimensional grid of voxels, of whether the tissue represented in the voxel is skeletal tissue.

Referring to block 246, in some embodiments, the visual representation of the skeleton excludes representations of respective groups of non-zero voxels in the three-dimensional grid of voxels having a total volume below a second volume threshold. In some embodiments, this simplifies the visual representation for the clinician, while still providing sufficient spatial context for the tumor position in the subject.

Referring to block 247, in some embodiments, method 200 includes displaying the visual representation of the tumor tissue and the visual representation of the skeleton of the subject in a single image (i) in a same spatial orientation as in the set of medical images, and (ii) at a same relative size as in the set of medical images. For example, radiology report 702 includes panel 706 showing three-dimensional representations of the tumor 710, as well as a partial skeleton 708 and trachea/primary bronchi 712 of the patient to provide a spatial perspective for the NSCLC tumor. Illustration of the tumor in context with non-cancerous tissue and the skeleton assist a physician in evaluating therapeutic options, including the viability of surgical intervention.

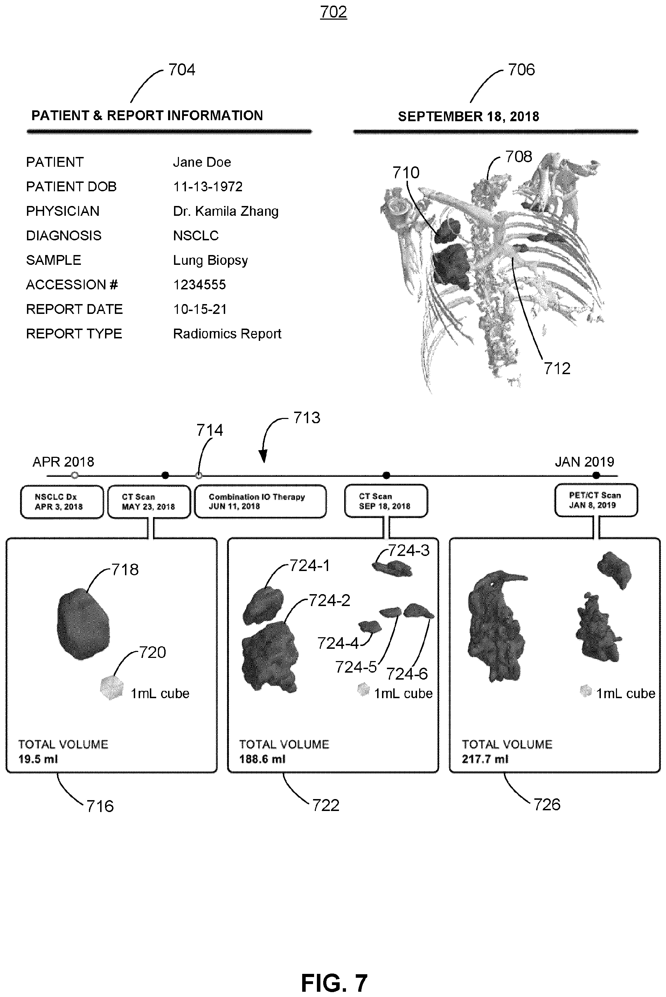

Referring to block 248, in some embodiments, method 200 includes generating a report comprising the single image comprising the visual representation of the tumor tissue and the visual representation of the skeleton of the subject. For example, each of radiology reports 702, 802, and 902, illustrated in Figures 7, 8, and 9, respectively, include a panel 706, 806, and 930, illustrating the tumor in context with a partial skeleton of the subject, aiding clinical evaluation of possible therapeutic interventions.

Referring to block 250, in some embodiments, the report further comprises one or more measurement for the cancerous tissue. For example, each of radiology reports 702, 802, and 902, illustrated in Figures 7, 8, and 9, respectively, include one or more measurements for the cancerous tissue. Report 702, for example, provides the total tumor volume for the cancer at each time point imaging data is available, in panels 716, 722, and 726. Report 802 provides additional tumor measurements, including various diameter measurements and density measurements for the tumor at each time point imaging data is available, in panels 816 and 818. Report 902 provides tumor volume and diameter measurements in panel 914. Providing common measurements for a cancerous tissue on a radiology report described herein simplifies the review and evaluation process for a clinician, who would otherwise need to generate these calculations themselves.

Referring to block 252, in some embodiments, the one or more measurement for the cancerous tissue comprises at least one measurement selected from a volume for the cancerous tissue, a cross-sectional length for the cancerous tissue, a density measurement for the cancerous tissue, a density for a non-cancerous tissue in the plurality of tissues, a distance from the cancerous tissue to a non-cancerous tissue in the plurality of tissues, and a distance from the cancerous tissue to a vascular structure in the three-dimensional region of interest of the subject. Referring to block 254, in some embodiments, the report further comprises a change in a measurement for the cancerous tissue over time. For example, a difference between a measurement for the tumor (e.g., tumor volume) at a first time corresponding to a first imaging analysis and a second time corresponding to a second imaging analysis. Reporting of a difference in a measurement allows for easy evaluation of the progression of a tumor without need for the clinician to make further measurements or calculations.

Referring to block 256, in some embodiments, the report further comprises, for each respective timepoint in a plurality of timepoints, a respective visual representation of the tumor tissue at the respective timepoint. For example, radiology reports 702 and 802 provide visual representations of respective tumors from multiple imaging evaluations in panels 716, 722, and 726 and panels 816 and 818, respectively.

Referring to block 258, in some embodiments, the report further comprises a timeline indicating the timing of one or more events associated with the cancer in the subject. For example, radiology report 702, shown in Figure 7, illustrates a relevant medical timeline 713 for the NSCLC in the patient, starting from the initial diagnosis date for the cancer (April 2018) through the last relevant medical event for the cancer (January 2019). The timeline includes points 714 for events such as the initial diagnosis, medical imaging analyses, and therapeutic interventions (e.g., the start of a particular therapy). The combined display of the timeline 713 with medical interventions and time series panels 716, 722, and 726, facilitate quick evaluation by the clinician. For example, it can be quickly recognized that the combination IO therapy initiated on June 11, 2018, did not halt the progression of the tumor, as it grew significantly from a total volume of 19.5 mL on May 23, 2018, to 188.6 mL on September 18, 2018.

Referring to block 260, in some embodiments, an event in the one or more events associated with the cancer in the subject is selected from an imaging examination of the cancer, a medical intervention for the cancer, a diagnosis for the cancer, a prognosis for the cancer, and a progression or regression of the cancer. Referring to block 262, in some embodiments, the report further comprises a prognosis for the cancer in the subject. In some embodiments, the prognosis is a predicted survival time. In some embodiments, the prognosis is a likelihood of survival for a defined time interval (e.g., 6-month, 12-month, 2-year, 5-year, or 10-year survival) In some embodiments, the prognosis is a predicted response to a therapy.

Referring to block 264, in some embodiments, the report is displayed in a user interface on a second computer system comprising one or more processors, memory coupled to the one or more processors, and a display. In some embodiments, the report is an interactive report. For example, report 902 illustrated in Figure 9 includes a plurality of user interface (UI) affordances 906, 908, 910, 912, etc., allowing a clinician to easily navigate between images of the patient's tumor at different periods of time.

Referring to block 266, in some embodiments, the single image further comprises a visual representation of a reference shape in the single image corresponding to a volume in the three-dimensional region of interest of the subject at a size of the same proportion to the visual representation of the tumor tissue and the visual representation of the skeleton of the subject relative to the set of medical images. For example, reports 702, 802, and 902 each include a visual representation of a reference volume to provide context for the size of a tumor, in panels 716, 722, and 726, panels 816 and 818, and panel 914, respectively.

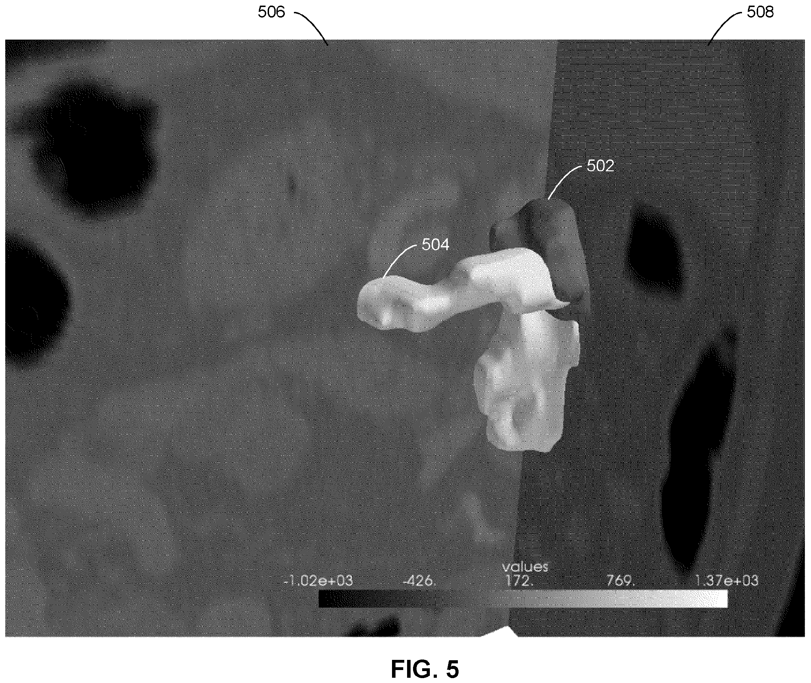

Referring to block 268, in some embodiments, the single image further comprises a visual representation of a reference axial, coronal, or sagittal slice of the three-dimensional region of interest of the subject from the first set of medical images. Referring to block 270, in some embodiments, the reference axial, coronal, or sagittal slice bisects the tumor tissue. For example, Figure 5 shows visual representations of a pancreatic tumor 502 and pancreas 504 bisected by coronal plane 506 and sagittal plane 508 from the CT scan. Co-display of a tumor representation and a reference axial, coronal, or sagittal slice of a medical imaging set provides additional context to a clinician evaluating possible therapeutic interventions.

Referring to block 272, in some embodiments, the single image comprises a three-dimensional representation of the tumor tissue and skeleton of the subject. Programs for generating three-dimensional representations are known in the art. For example, PyVista is a helper library for the Visualization Toolkit (VTK) open source software.

Referring to block 274, in some embodiments, the single image comprises a two-dimensional representation of the tumor tissue and skeleton of the subject. Programs for generating two-dimensional representations are known in the art. In addition to VTK, Matplotlib can be used to generate two-dimensional representations. See, for example, Hunter, J.D., "Matplotlib: A 2D graphics environment," Computing in Science \& Engineering, 9(3):90-95 (2007), the disclosure of which is incorporated herein by reference.

Referring to block 276, in some embodiments, the single image further comprises a visual representation of the non-cancerous tissue of the subject, in the same spatial orientation as in the set of medical images, and at a same relative size as in the set of medical images.

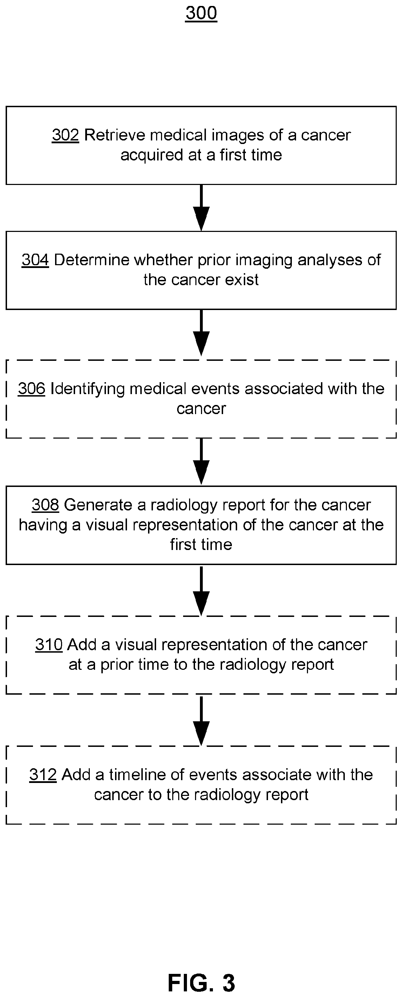

Figure 3 likewise illustrates a method for visualizing a cancer in a subject. The method is performed at a computer system comprising one or more processors and a memory coupled to the one or more processors, the memory comprising one or more programs configured to be executed by the one or more processors.

Method 300 includes retrieving (302) a first set of medical images of a three-dimensional region of interest of the subject, from a most recent medical imaging evaluation of the cancer performed at a first time point, from a first medical database. In some embodiments, the retrieving is in response to receiving a request for a radiology report for a cancer in a subject, e.g., made by a clinician. In other embodiments, the retrieving is automated by the system, e.g., according to a schedule for generating radiology reports following medical imaging evaluations.

In some embodiments, method 300 also includes querying (304) a second medical database to identify one or more medical imaging evaluations of the cancer performed prior to the first time point. In some embodiments, the second medical database is the same medical database as the first medical database, e.g., an electronic repository of medical imaging data or a medical record for the patient. In some embodiments, the second medical database is different than the first medical database.

Method 300 includes generating (306) a radiology report for the cancer in the subject comprising a first image displaying a visual representation of the cancer and a visual representation of a least a portion of a skeleton for the subject based on the first set of medical images. In some embodiments, the first image further comprises a visual representation of a non-cancerous tissue of the subject based on the first set of medical images. In some embodiments, the first set of medical images comprises a computed tomography (CT) scan of the three-dimensional region of the subject.

In some embodiments, method 300 includes retrieving information for one or more medical imaging evaluations performed prior to the first time point from the second medical database, and adding (310) a second image to the radiology report. The second image displaying a visual representation of the cancer based on a respective medical imaging evaluation of the one or more medical imaging evaluations performed prior to the first time point. In some embodiments, the second image further comprises a reference shape corresponding to a volume in the three-dimensional region of interest of the subject.

In some embodiments, method 300 includes identifying (306), from a medical record for the subject, one or more events associated with the cancer in the subject, and adding (312) a timeline to the radiology report, the timeline comprising an indication of the corresponding date for each respective event in the one or more events associated with the cancer in the subject. In some embodiments, a respective event in the one or more events associated with the cancer in the subject is selected from an initial diagnosis, a medical imaging evaluation, and a therapeutic intervention.

In some embodiments, the first image is generated by a process that includes segmenting the first set of medical images by assigning, for each respective set of one or more pixels in a first plurality of sets of one or more pixels in the set of medical images, a label corresponding to a respective tissue type in the plurality of tissue types based on one or more corresponding pixel values for the respective set of one or more pixels, e.g., as described herein above. In some embodiments, the process also includes generating, for each respective tissue type in the plurality of tissue types, a corresponding mask based on respective sets of one or more pixels assigned the label corresponding to the respective tissue type, e.g., as described herein above. In some embodiments, the process also includes generating a visual representation of the tumor tissue of the subject based on the corresponding mask for the tumor tissue, e.g., as described herein above. In some embodiments, the process also includes generating a visual representation of a skeleton of the subject within the region of interest based on the first set of medical images, e.g., as described herein above. In some embodiments, the process also includes displaying the visual representation of the tumor tissue and the visual representation of the skeleton of the subject in a single image, in a same spatial orientation as in the set of medical images, and at a same relative size as in the set of medical images, e.g., as described herein above.

In some embodiments, generating the visual representation of the tumor tissue comprises generating a corresponding mesh surface for the tumor tissue based on an outer boundary of the corresponding mask for the tumor tissue and smoothing edges of the corresponding mesh surface for the tumor tissue.

In some embodiments, method 300 also includes determining a volume for the cancer in the subject based on a volume contained within the mesh surface for the tumor tissue. In some embodiments, the volume is determined after smoothing edges of the corresponding mesh surface for the tumor tissue.

In some embodiments, generating the visual representation of the skeleton includes determining, for each respective set of one or more pixels in a second plurality of sets of one or more pixels in the set of medical images, a corresponding tissue density, e.g., as described herein above. In some embodiments, it also includes generating a corresponding mask for the skeleton based on respective sets of one or more pixels in the second plurality of sets of one or more pixels having a tissue density satisfying a set of one or more bone density criteria, e.g., as described herein above. In some embodiments, it also includes generating a corresponding mesh surface for the skeleton based on an outer boundary of the corresponding mask for the skeleton.

In some embodiments, the first set of medical images comprises a computed tomography (CT) scan and the set of one or more bone density criteria comprise a first criterion that is satisfied by a measure of central tendency for the pixel intensity of the respective set of one or more pixels of greater than a first threshold of from 300 to 500 Hounsfield units (HUs).

In some embodiments, the first set of medical images comprises a computed tomography (CT) scan and the set of one or more bone density criteria comprise a second criterion that is satisfied by a measure of central tendency for the pixel intensity of the respective set of one or more pixels of less than greater than a second threshold of from 1750 to 2500 Hounsfield units (HUs).

### Example Implementations

### Figure 7 - NSCLC Report with a Timeline and Time Series of the Tumor

Figure 7 illustrates an example radiology report generated using the methods and systems described herein, in accordance with some embodiments. Specifically, Figure 7 illustrates a radiology report 702 for patient Jane Doe who has a non-small cell lung cancer (NSCLC). The report includes biographical information 704 about the patient, e.g., retrieved from a medical record 47. The biographical information provides the physician with targeted information relevant to clinical treatment decisions, such as the patient's age/date of birth, a previous diagnosis for the cancer, biographical information for the medical imaging file from which the visualization 706 of the tumor was prepared, and the date the report was generated.

The report 702 also includes a panel 706 showing three-dimensional representations of the tumor 710, as well as a partial skeleton 708 and trachea/primary bronchi 712 of the patient to provide a spatial perspective for the NSCLC tumor. Report 702 also illustrates a relevant medical timeline 713 for the NSCLC in the patient, starting from the initial diagnosis date for the cancer (April 2018) through the last relevant medical event for the cancer (January 2019). The timeline includes points 714 for events such as the initial diagnosis, medical imaging analyses, and therapeutic interventions (e.g., the start of a particular therapy).

The report 702 also includes time series images 716, 722, and 726 of visual representations (718, 724, etc.) of the NSCLC, for visualizing the progression of the cancer over time. Each image is generated from a different medical imaging evaluation, as indicated immediately above the image. In order to facilitate quick evaluation of the cancer progression, the time series images do not include visual representations of the skeleton of the subject or of a proximal non-cancerous tissue (e.g., trachea/primary bronchi). However, the time series images show a reference volume 720 to provide perspective as to the size of the tumor, as well as a total volume of the tumor islands in the bottom left of the panel. In some embodiments, a time series of images include a visual representation of a skeleton of the subject and/or one or more non-cancerous tissues / organs.

The combined display of the timeline 713 with medical interventions and time series panels 716, 722, and 726, facilitate quick evaluation by the clinician. For example, it can be quickly recognized that the combination IO therapy initiated on June 11, 2018, did not halt the progression of the tumor, as it grew significantly from a total volume of 19.5 mL on May 23, 2018, to 188.6 mL on September 18, 2018. The tumor then continued to grow to a volume of 217.7 mL on January 8, 2019.

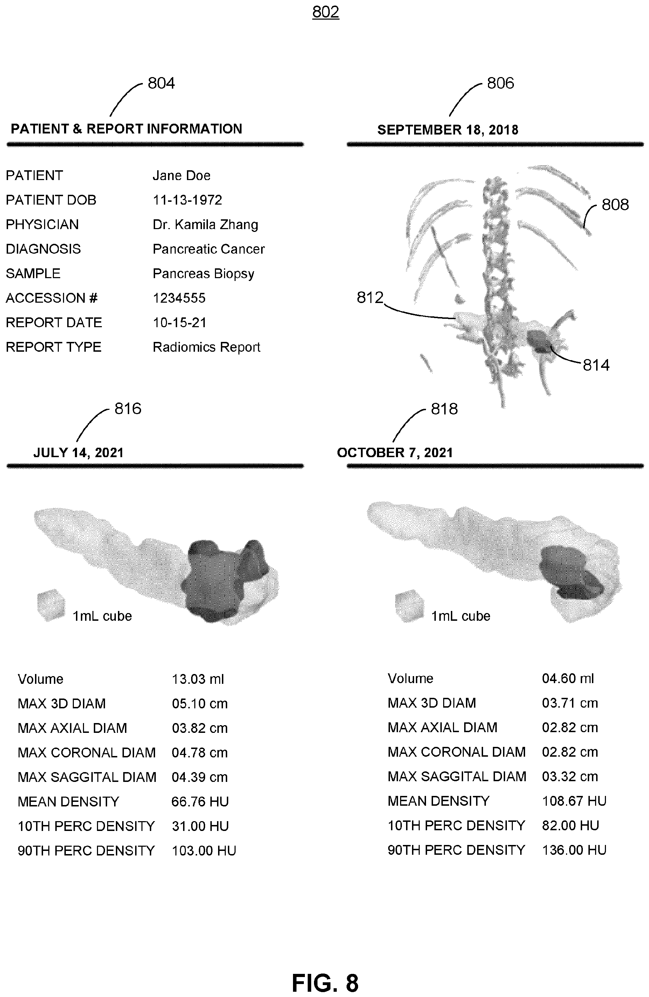

### Figure 8 - Pancreatic Cancer Report with a Time Series of the Tumor

Figure 8 illustrates another example radiology report generated using the methods and systems described herein, in accordance with some embodiments. Specifically, Figure 8 illustrates a radiology report 802 for patient Jane Doe who has pancreatic cancer. The report includes biographical information 804 about the patient, e.g., retrieved from a medical record 47. The biographical information provides the physician with targeted information relevant to clinical treatment decisions, such as the patient's age/date of birth, a previous diagnosis for the cancer, biographical information for the medical imaging file from which the visualization 806 of the tumor was prepared, and the date the report was generated.

The report 802 also includes a single image 806 showing three-dimensional representations of the tumor 814, as well as a partial skeleton 808 and pancreas 812 of the patient to provide a spatial perspective for the tumor. Unlike report 702, report 802 does not include a relevant medical timeline. However, report 802 does include side by side images 816 and 818 of the pancreatic cancer and pancreas at two time points, along with a reference cube for size perspective. Images 816 and 818 also provide several measured characteristics of the cancer, including volume, max diameter, max axial diameter, max coronal diameter, max saggital diameter, mean tumor density, 10^{th} percentile tumor density, and 90^{th} percentile tumor density.

The side by side images 816 and 818 of the pancreatic cancer and pancreas at two time points facilitate quick evaluation by the clinician. For example, it can be quickly recognized that the cancer is responding to the therapeutic regime, as the tumor shrunk in volume from 13 mL in July, 2021, to 4.6 mL in October, 2021, as well as in every other reported size metric.

### Figure 9 - Non-Small Cell Lung Cancer (NSCLC) Report with a Time Series of the Tumor

Figure 9 illustrates another example radiology report generated using the methods and systems described herein, in accordance with some embodiments. Specifically, Figure 9 illustrates an interactive radiology report 902, displayed on a computer. The interactive radiology report includes a plurality of user interface (UI) affordances 906, 908, 910, 912, etc., allowing a clinician to easily navigate between images of the patient's tumor at different periods of time. For example, the interactive radiology report 902 as illustrated in Figure 9 displays images of a NSCLC tumor from a medical imaging evaluation performed on October 7, 2021, as indicated by the bolding and underlining of affordance 910. By selecting another one of the UI affordances, a user can navigate to an image of the tumor at a different point in time. For example, by selecting affordance 908, the computer will replace display of the current user interface with a user interface showing one a visual representation of the tumor as generated from a medical imaging evaluation performed on July 14, 2021, providing the user with information on how the tumor has progressed between July 14, 2021, and October 7, 2021. Selecting UI affordance 906 would navigate to a user interface showing a visual representation of the tumor as of the medical imaging evaluation that immediately preceded the medical imaging evaluation currently being displayed. Likewise, selecting UI affordance 912 would navigate to a user interface showing a visual representation of the tumor as of the medical imaging evaluation that immediately followed the medical imaging evaluation currently being displayed.

The report 902 includes a panel 914 showing three-dimensional visual representations of three tumor islands 918, 920, and 922, along with a reference volume 920 for perspective as to the size of the tumor islands. The panel 914 also provide several measured characteristics of each tumor island, including volume, max diameter, max axial diameter, max coronal diameter, and max saggital diameter, for quick reference by a clinician. The report 902 also includes a panel 930 showing a three-dimensional representation of the tumor 934 as well as a partial skeleton 932 of the patient to provide a spatial perspective for the tumor. While the information and visuals illustrated in Figure 9 do not include any information on the direct comparison of the tumor or characteristics of the tumor over time, in some embodiments, one or more such comparison is added to the user interface display to further facilitate comparison of the tumor over time.

The easy navigation between representations of the tumor from different medical imaging evaluations facilitate quick evaluation by the clinician. For example, it can be quickly recognized that the cancer is responding or not responding to a therapy over time.

Plural instances may be provided for components, operations or structures described herein as a single instance. Finally, boundaries between various components, operations, and data stores are somewhat arbitrary, and particular operations are illustrated in the context of specific illustrative configurations. Other forms of functionality are envisioned and may fall within the scope of the implementation(s). In general, structures and functionality presented as separate components in the example configurations may be implemented as a combined structure or component. Similarly, structures and functionality presented as a single component may be implemented as separate components. These and other variations, modifications, additions, and improvements fall within the scope of the implementation(s).

It will also be understood that, although the terms "first," "second," etc. may be used herein to describe various elements, these elements should not be limited by these terms. These terms are only used to distinguish one element from another. For example, a first attribute could be termed a second attribute, and, similarly, a second attribute could be termed a first attribute, without changing the meaning of the description, so long as all occurrences of the "first attribute" are renamed consistently and all occurrences of the "second attribute" are renamed consistently. The first attribute, and the second attribute are both attributes, but they are not the same attribute, unless specified otherwise.

The terminology used herein is for the purpose of describing particular implementations only and is not intended to be limiting of the claims. As used in the description of the implementations and the appended claims, the singular forms "a", "an" and "the" are intended to include the plural forms as well, unless the context clearly indicates otherwise. It will also be understood that the term "and/or" as used herein refers to and encompasses any and all possible combinations of one or more of the associated listed items. It will be further understood that the terms "comprises" and/or "comprising," when used in this specification, specify the presence of stated features, integers, steps, operations, elements, and/or components, but do not preclude the presence or addition of one or more other features, integers, steps, operations, elements, components, and/or groups thereof.

As used herein, the term "if" may be construed to mean "when" or "upon" or "in response to determining" or "in accordance with a determination" or "in response to detecting," that a stated condition precedent is true, depending on the context. Similarly, the phrase "if it is determined (that a stated condition precedent is true)" or "if (a stated condition precedent is true)" or "when (a stated condition precedent is true)" may be construed to mean "upon determining" or "in response to determining" or "in accordance with a determination" or "upon detecting" or "in response to detecting" that the stated condition precedent is true, depending on the context.

The foregoing description included example systems, methods, techniques, instruction sequences, and computing machine program products that embody illustrative implementations. For purposes of explanation, numerous specific details were set forth in order to provide an understanding of various implementations of the inventive subject matter. It will be evident, however, to those skilled in the art that implementations of the inventive subject matter may be practiced without these specific details. In general, well-known instruction instances, protocols, structures and techniques have not been shown in detail.

The foregoing description, for purpose of explanation, has been described with reference to specific implementations. However, the illustrative discussions above are not intended to be exhaustive or to limit the implementations to the precise forms disclosed. Many modifications and variations are possible in view of the above teachings. The implementations were chosen and described in order to best explain the principles and their practical applications, to thereby enable others skilled in the art to best utilize the implementations and various implementations with various modifications as are suited to the particular use contemplated.

### Disclosed Items:

1. A method for visualizing a cancer in a subject, the method comprising:
   at a computer system comprising one or more processors and a memory coupled to the one or more processors, the memory comprising one or more programs configured to be executed by the one or more processors, wherein the one or more programs comprise instructions for:
   A) obtaining a first set of medical images of a three-dimensional region of interest of the subject, wherein:
      the first set of medical images was collected at a first time using a first medical imaging modality,
      the three-dimensional region of interest of the subject comprises a plurality of tissue types, and
      the plurality of tissue types comprises a tumor tissue and a non-cancerous tissue of the subject;
   B) segmenting the first set of medical images by assigning, for each respective set of one or more pixels in a first plurality of sets of one or more pixels in the set of medical images, a label corresponding to a respective tissue type in the plurality of tissue types based on one or more corresponding pixel values for the respective set of one or more pixels;
   C) generating, for each respective tissue type in the plurality of tissue types, a corresponding mask based on respective sets of one or more pixels assigned the label corresponding to the respective tissue type;
   D) generating a visual representation of the tumor tissue of the subject based on the corresponding mask for the tumor tissue;
   E) generating a visual representation of a skeleton of the subject within the region of interest based on the first set of medical images; and
   F) displaying the visual representation of the tumor tissue and the visual representation of the skeleton of the subject in a single image, in a same spatial orientation as in the set of medical images, and at a same relative size as in the set of medical images.
2. The method of item 1, wherein the first set of medical images comprises a computed tomography (CT) scan of the three-dimensional region of the subject.
3. The method of item 1 or 2, wherein the cancer is a non-small cell lung cancer (NSCLC) and the plurality of tissue types includes non-cancerous lung tissue.
4. The method of item 1 or 2, wherein the cancer is a pancreatic cancer and the plurality of tissue types includes non-cancerous pancreatic tissue.
5. The method of any one of items 1-4, wherein the plurality of tissue types includes lymph tissue.
6. The method of any one of items 1-5, wherein the plurality of tissue types includes vascular tissue.
7. The method of any one of items 1-6, wherein generating the visual representation of the tumor tissue comprises generating a corresponding mesh surface for the tumor tissue based on an outer boundary of the corresponding mask for the tumor tissue and smoothing edges of the corresponding mesh surface for the tumor tissue.
8. The method of any one of items 1-7, wherein each respective set of one or more pixels in the first plurality of sets of one or more pixels corresponds to a respective voxel in a uniform three-dimensional grid of voxels defined for the set of medical images; and
   for a respective tissue type in the plurality of tissue types, the corresponding mask comprises a binary indication, for each respective voxel in the three-dimensional grid of voxels, of whether the tissue represented in the voxel is the respective tissue type.
9. The method of item 8, wherein the corresponding mask for the tumor tissue comprises a plurality of groups of non-zero voxels, wherein each respective group of non-zero voxels in the plurality of groups of non-zero voxels is separated from every other respective group of non-zero voxels in the plurality of groups of non-zero voxels by at least one zero voxel.
10. The method of item 9, wherein generating the visual representation of the tumor tissue comprises generating a corresponding mesh surface for each respective group of non-zero voxels in the plurality of groups of non-zero voxels.
11. The method of item 9 or 10, wherein the visual representation of the tumor tissue excludes representations of respective groups of non-zero voxels having a total volume below a first volume threshold.
12. The method of any one of items 7-11, further comprising determining a volume for the cancer in the subject based on a volume contained within the mesh surface for the tumor tissue.
13. The method of item 12, wherein the volume is determined after smoothing edges of the corresponding mesh surface for the tumor tissue.
14. The method of any one of items 1-13, wherein generating the visual representation of the skeleton comprises:
   determining, for each respective set of one or more pixels in a second plurality of sets of one or more pixels in the set of medical images, a corresponding tissue density; and
   generating a corresponding mask for the skeleton based on respective sets of one or more pixels in the second plurality of sets of one or more pixels having a tissue density satisfying a set of one or more bone density criteria; and
   generating a corresponding mesh surface for the skeleton based on an outer boundary of the corresponding mask for the skeleton.
15. The method of item 14, wherein the first set of medical images comprises a computed tomography (CT) scan and the set of one or more bone density criteria comprise a first criterion that is satisfied by a measure of central tendency for the pixel intensity of the respective set of one or more pixels of greater than a first threshold of from 300 to 500 Hounsfield units (HUs).
16. The method of item 14 or 15, wherein the first set of medical images comprises a computed tomography (CT) scan and the set of one or more bone density criteria comprise a second criterion that is satisfied by a measure of central tendency for the pixel intensity of the respective set of one or more pixels of less than greater than a second threshold of from 1750 to 2500 Hounsfield units (HUs).
17. The method of any one of items 14-16, further comprising smoothing edges of the corresponding mesh surface for the skeleton.
18. The method of any one of items 14-17, wherein each respective set of one or more pixels in the second plurality of sets of one or more pixels corresponds to a respective voxel in a second three-dimensional grid of voxels defined for the set of medical images; and
   the corresponding mask for the skeleton comprises a binary indication, for each respective voxel in the second three-dimensional grid of voxels, of whether the tissue represented in the voxel is skeletal tissue.
19. The method of item 18, wherein the visual representation of the skeleton excludes representations of respective groups of non-zero voxels in the three-dimensional grid of voxels having a total volume below a second volume threshold.
20. The method of any one of items 1-19, wherein the displaying F) comprises generating a report comprising the single image comprising the visual representation of the tumor tissue and the visual representation of the skeleton of the subject.
21. The method of any one of items 1-20, wherein the single image further comprises a visual representation of a reference shape in the single image corresponding to a volume in the three-dimensional region of interest of the subject at a size of the same proportion to the visual representation of the tumor tissue and the visual representation of the skeleton of the subject relative to the set of medical images.
22. The method of any one of items 1-21, wherein the single image further comprises a visual representation of a reference axial, coronal, or sagittal slice of the three-dimensional region of interest of the subject from the first set of medical images.
23. The method of item 22, wherein the reference axial, coronal, or sagittal slice bisects the tumor tissue.
24. The method of any one of items 20-22, wherein the report further comprises one or more measurement for the cancerous tissue.
25. The method of item 24, wherein the one or more measurement for the cancerous tissue comprises at least one measurement selected from:
   a volume for the cancerous tissue,
   a cross-sectional length for the cancerous tissue,
   a density measurement for the cancerous tissue,
   a density for a non-cancerous tissue in the plurality of tissues,
   a distance from the cancerous tissue to a non-cancerous tissue in the plurality of tissues, and
   a distance from the cancerous tissue to a vascular structure in the three-dimensional region of interest of the subject.
26. The method of any one of items 20-25, wherein the report further comprises a change in a measurement for the cancerous tissue over time.
27. The method of any one of items 20-26, wherein the report further comprises, for each respective timepoint in a plurality of timepoints, a respective visual representation of the tumor tissue at the respective timepoint.
28. The method of any one of items 20-27, wherein the report further comprises a timeline indicating the timing of one or more events associated with the cancer in the subject.
29. The method of item 28, wherein an event in the one or more events associated with the cancer in the subject is selected from:
   an imaging examination of the cancer,
   a medical intervention for the cancer,
   a diagnosis for the cancer,
   a prognosis for the cancer, and
   a progression or regression of the cancer.
30. The method of any one of items 20-29, wherein the report further comprises a prognosis for the cancer in the subject.
31. The method of any one of items 20-30, wherein the report is displayed in a user interface on a second computer system comprising one or more processors, memory coupled to the one or more processors, and a display.
32. The method of any one of items 1-31, wherein the single image comprises a three-dimensional representation of the tumor tissue and skeleton of the subject.
33. The method of any one of items 1-31, wherein the single image comprises a two-dimensional representation of the tumor tissue and skeleton of the subject.
34. The method of any one of items 1-33, wherein the single image further comprises a visual representation of the non-cancerous tissue of the subject, in the same spatial orientation as in the set of medical images, and at a same relative size as in the set of medical images.
35. A method for visualizing a cancer in a subject, the method comprising:
   at a computer system comprising one or more processors and a memory coupled to the one or more processors, the memory comprising one or more programs configured to be executed by the one or more processors,
   responsive to receiving a request for a radiology report for a cancer in a subject:
   A) retrieving a first set of medical images of a three-dimensional region of interest of the subject, from a most recent medical imaging evaluation of the cancer performed at a first time point, from a first medical database;
   B) querying a second medical database to identify one or more medical imaging evaluations of the cancer performed prior to the first time point; and
   C) generating a radiology report for the cancer in the subject comprising a first image displaying a visual representation of the cancer and a visual representation of a least a portion of a skeleton for the subject based on the first set of medical images.
36. The method of item 35, wherein the first image further comprises a visual representation of a non-cancerous tissue of the subject based on the first set of medical images.
37. The method of item 35 or 36, further comprising:
   retrieving information for one or more medical imaging evaluations performed prior to the first time point from the second medical database; and
   adding a second image to the radiology report, the second image displaying a visual representation of the cancer based on a respective medical imaging evaluation of the one or more medical imaging evaluations performed prior to the first time point.
38. The method of item 37, wherein the second image further comprises a reference shape corresponding to a volume in the three-dimensional region of interest of the subject.
39. The method of any one of items 35-38, further comprising:
   identifying, from a medical record for the subject, one or more events associated with the cancer in the subject; and
   adding a timeline to the radiology report, the timeline comprising an indication of the corresponding date for each respective event in the one or more events associated with the cancer in the subject.
40. The method of item 39, wherein a respective event in the one or more events associated with the cancer in the subject is selected from an initial diagnosis, a medical imaging evaluation, and a therapeutic intervention.
41. The method of any one of items 35-40, wherein the first image is generated by a process comprising:
   i) segmenting the first set of medical images by assigning, for each respective set of one or more pixels in a first plurality of sets of one or more pixels in the set of medical images, a label corresponding to a respective tissue type in the plurality of tissue types based on one or more corresponding pixel values for the respective set of one or more pixels;
   ii) generating, for each respective tissue type in the plurality of tissue types, a corresponding mask based on respective sets of one or more pixels assigned the label corresponding to the respective tissue type;
   iii) generating a visual representation of the tumor tissue of the subject based on the corresponding mask for the tumor tissue;
   iv) generating a visual representation of a skeleton of the subject within the region of interest based on the first set of medical images; and
   v) displaying the visual representation of the tumor tissue and the visual representation of the skeleton of the subject in a single image, in a same spatial orientation as in the set of medical images, and at a same relative size as in the set of medical images.
42. The method of any one of items 35-41, wherein generating the visual representation of the tumor tissue comprises generating a corresponding mesh surface for the tumor tissue based on an outer boundary of the corresponding mask for the tumor tissue and smoothing edges of the corresponding mesh surface for the tumor tissue.
43. The method of item 42, further comprising determining a volume for the cancer in the subject based on a volume contained within the mesh surface for the tumor tissue.
44. The method of item 43, wherein the volume is determined after smoothing edges of the corresponding mesh surface for the tumor tissue.
45. The method of any one of items 41-44, wherein generating the visual representation of the skeleton comprises:
   determining, for each respective set of one or more pixels in a second plurality of sets of one or more pixels in the set of medical images, a corresponding tissue density; and
   generating a corresponding mask for the skeleton based on respective sets of one or more pixels in the second plurality of sets of one or more pixels having a tissue density satisfying a set of one or more bone density criteria; and
   generating a corresponding mesh surface for the skeleton based on an outer boundary of the corresponding mask for the skeleton.
46. The method of item 45, wherein the first set of medical images comprises a computed tomography (CT) scan and the set of one or more bone density criteria comprise a first criterion that is satisfied by a measure of central tendency for the pixel intensity of the respective set of one or more pixels of greater than a first threshold of from 300 to 500 Hounsfield units (HUs).
47. The method of item 46, wherein the first set of medical images comprises a computed tomography (CT) scan and the set of one or more bone density criteria comprise a second criterion that is satisfied by a measure of central tendency for the pixel intensity of the respective set of one or more pixels of less than greater than a second threshold of from 1750 to 2500 Hounsfield units (HUs).
48. The method of any one of items 35-47, wherein the first set of medical images comprises a computed tomography (CT) scan of the three-dimensional region of the subject.
49. A computer system, comprising:
   one or more processors; and
   memory storing one or more programs, the one or more programs including instructions for performing the method of any of items 1-48.
50. A non-transitory computer-readable storage medium storing one or more storing one or more programs for execution by a computer system with one or more processors, the one or more programs including instructions for performing the method of any of items 1-48.

## Claims

1. A method for visualizing a cancer in a subject, the method comprising:
at a computer system comprising one or more processors and a memory coupled to the one or more processors, the memory comprising one or more programs configured to be executed by the one or more processors, wherein the one or more programs comprise instructions for:
A) obtaining a first set of medical images of a three-dimensional region of interest of the subject, wherein:
the first set of medical images was collected at a first time using a first medical imaging modality,
the three-dimensional region of interest of the subject comprises a plurality of tissue types, and
the plurality of tissue types comprises a tumor tissue and a non-cancerous tissue of the subject;
B) segmenting the first set of medical images by assigning, for each respective set of one or more pixels in a first plurality of sets of one or more pixels in the set of medical images, a label corresponding to a respective tissue type in the plurality of tissue types based on one or more corresponding pixel values for the respective set of one or more pixels;
C) generating, for each respective tissue type in the plurality of tissue types, a corresponding mask based on respective sets of one or more pixels assigned the label corresponding to the respective tissue type;
D) generating a visual representation of the tumor tissue of the subject based on the corresponding mask for the tumor tissue;
E) generating a visual representation of a skeleton of the subject within the region of interest based on the first set of medical images; and
F) displaying the visual representation of the tumor tissue relative to the visual representation of the skeleton of the subject in a single image, in a same spatial orientation as in the set of medical images, and at a same relative size as in the set of medical images, wherein the combined visual representation of the tumor tissue and the skeleton provides spatial context for the tumor tissue, thereby informing treatment and/or surgical intervention decisions for the subject.

2. The method of claim 1, wherein the first set of medical images comprises a computed tomography (CT) scan of the three-dimensional region of the subject.

3. The method of claim 1 or 2, wherein
the cancer is a non-small cell lung cancer (NSCLC) and the plurality of tissue types includes non-cancerous lung tissue,
the cancer is a pancreatic cancer and the plurality of tissue types includes non-cancerous pancreatic tissue,
the plurality of tissue types includes lymph tissue, or
the plurality of tissue types includes vascular tissue.

4. The method of any one of claims 1-3, wherein generating the visual representation of the tumor tissue comprises generating a corresponding mesh surface for the tumor tissue based on an outer boundary of the corresponding mask for the tumor tissue and smoothing edges of the corresponding mesh surface for the tumor tissue.

5. The method of any one of claims 1-4, wherein each respective set of one or more pixels in the first plurality of sets of one or more pixels corresponds to a respective voxel in a uniform three-dimensional grid of voxels defined for the set of medical images; and
for a respective tissue type in the plurality of tissue types, the corresponding mask comprises a binary indication, for each respective voxel in the three-dimensional grid of voxels, of whether the tissue represented in the voxel is the respective tissue type,
optionally wherein the corresponding mask for the tumor tissue comprises a plurality of groups of non-zero voxels, wherein each respective group of non-zero voxels in the plurality of groups of non-zero voxels is separated from every other respective group of non-zero voxels in the plurality of groups of non-zero voxels by at least one zero voxel,
optionally wherein generating the visual representation of the tumor tissue comprises generating a corresponding mesh surface for each respective group of non-zero voxels in the plurality of groups of non-zero voxels,
and optionally wherein the visual representation of the tumor tissue excludes representations of respective groups of non-zero voxels having a total volume below a first volume threshold.

6. The method of claim 4 or 5, further comprising determining a volume for the cancer in the subject based on a volume contained within the mesh surface for the tumor tissue, wherein the volume is optionally determined after smoothing edges of the corresponding mesh surface for the tumor tissue.

7. The method of any one of claims 1-6, wherein generating the visual representation of the skeleton comprises:
determining, for each respective set of one or more pixels in a second plurality of sets of one or more pixels in the set of medical images, a corresponding tissue density; and
generating a corresponding mask for the skeleton based on respective sets of one or more pixels in the second plurality of sets of one or more pixels having a tissue density satisfying a set of one or more bone density criteria; and
generating a corresponding mesh surface for the skeleton based on an outer boundary of the corresponding mask for the skeleton, optionally
wherein the first set of medical images comprises a computed tomography (CT) scan and the set of one or more bone density criteria comprise a first criterion that is satisfied by a measure of central tendency for the pixel intensity of the respective set of one or more pixels of greater than a first threshold of from 300 to 500 Hounsfield units (HUs),
and/or a second criterion that is satisfied by a measure of central tendency for the pixel intensity of the respective set of one or more pixels of less than greater than a second threshold of from 1750 to 2500 Hounsfield units (HUs).

8. The method of claim 7, wherein each respective set of one or more pixels in the second plurality of sets of one or more pixels corresponds to a respective voxel in a second three-dimensional grid of voxels defined for the set of medical images; and
the corresponding mask for the skeleton comprises a binary indication, for each respective voxel in the second three-dimensional grid of voxels, of whether the tissue represented in the voxel is skeletal tissue,
optionally wherein the visual representation of the skeleton excludes representations of respective groups of non-zero voxels in the three-dimensional grid of voxels having a total volume below a second volume threshold.

9. The method of any one of claims 1-8 wherein the displaying F) comprises generating a report comprising the single image comprising the visual representation of the tumor tissue and the visual representation of the skeleton of the subject.

10. The method of any one of claims 1-9, wherein the single image further comprises a visual representation of a reference shape in the single image corresponding to a volume in the three-dimensional region of interest of the subject at a size of the same proportion to the visual representation of the tumor tissue and the visual representation of the skeleton of the subject relative to the set of medical images.

11. The method of any one of claims 1-10, wherein the single image further comprises a visual representation of a reference axial, coronal, or sagittal slice of the three-dimensional region of interest of the subject from the first set of medical images,
optionally wherein the reference axial, coronal, or sagittal slice bisects the tumor tissue,
and optionally wherein the report further comprises one or more measurement for the cancerous tissue,
optionally wherein the one or more measurement for the cancerous tissue comprises at least one measurement selected from:
a volume for the cancerous tissue,
a cross-sectional length for the cancerous tissue,
a density measurement for the cancerous tissue,
a density for a non-cancerous tissue in the plurality of tissues,
a distance from the cancerous tissue to a non-cancerous tissue in the plurality of tissues, and
a distance from the cancerous tissue to a vascular structure in the three-dimensional region of interest of the subject.

12. The method of any one of claim 9-11, wherein the report further comprises
a change in a measurement for the cancerous tissue over time
for each respective timepoint in a plurality of timepoints, a respective visual representation of the tumor tissue at the respective timepoint, or
a timeline indicating the timing of one or more events associated with the cancer in the subject,
optionally wherein an event in the one or more events associated with the cancer in the subject is selected from:
an imaging examination of the cancer,
a medical intervention for the cancer,
a diagnosis for the cancer,
a prognosis for the cancer, and
a progression or regression of the cancer.

13. The method of any one of claims 9-12, wherein the report further comprises a prognosis for the cancer in the subject.

14. The method of any one of claims 1-13, wherein the single image comprises a two-dimensional or three-dimensional representation of the tumor tissue and skeleton of the subject.

15. The method of any one of claims 1-14, wherein the single image further comprises a visual representation of the non-cancerous tissue of the subject, in the same spatial orientation as in the set of medical images, and at a same relative size as in the set of medical images.

16. A computer system, comprising:
one or more processors; and
memory storing one or more programs, the one or more programs including instructions for performing the method of any of items 1-15.

17. A non-transitory computer-readable storage medium storing one or more storing one or more programs for execution by a computer system with one or more processors, the one or more programs including instructions for performing the method of any of items 1-15.
